(19) Europäisches Patentamt
European Patent Office
Office européen des brevets

(11) Veröffentlichungsnummer: **0 125 208**
**A1**

# (12) EUROPÄISCHE PATENTANMELDUNG

(21) Anmeldenummer: 84810205.9

(22) Anmeldetag: 30.04.84

(51) Int. Cl.³: C 07 D 499/00
A 61 K 31/43
//C07F7/18, C07F9/65,
C07D205/08

(30) Priorität: 06.05.83 CH 2491/83
02.09.83 CH 4831/83

(43) Veröffentlichungstag der Anmeldung:
14.11.84 Patentblatt 84/46

(84) Benannte Vertragsstaaten:
AT BE CH DE FR GB IT LI LU NL SE

(71) Anmelder: CIBA-GEIGY AG
Postfach
CH-4002 Basel(CH)

(72) Erfinder: Lang, Marc, Dr.
Rue des Ormes 6
F-68170 Rixheim(FR)

(54) **Aminoniederalkyl-penem-Verbindungen, Verfahren zu ihrer Herstellung, pharmazeutische Präparate, welche diese Verbindungen enthalten, und Verwendung von letzteren.**

(57) 2-Aminoniederalkyl-2-penem-Verbindungen der Formel

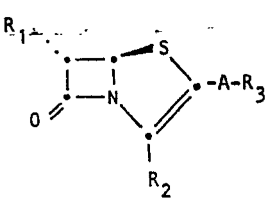

(I),

worin $R_1$ durch Hydroxy oder geschütztes Hydroxy substituiertes Niederalkyl ist, $R_2'$ Carboxyl oder geschütztes Carboxyl $R_2'$ darstellt, $R_3$ Amino, duch Niederalkyl substituiertes Amino, substituiertes Methylenamino oder geschütztes Amino bedeutet und A durch Niederalkyl substituiertes geradkettiges Niederalkylen ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat un $R_3$ Amino oder geschütztes Amino darstellt, Salze von solchen verbindungen der Formel I, die eine salzbildende Gruppe aufweisen, optische Isomer von Verbindungen der Formel I und Mischungen dieser optischen Isomere besitzen antibiotishe Eigenschaften. Die neuen Verbindungen könne z.B. in Form von antibiotisch wirksamen Präparaten zur Behandlung von Infektionskrankheiten verwendet werden. Die neuen Verbindungen können in an sich bekannter Weise hergestellt werden.

EP 0 125 208 A1

0125208



CIBA-GEIGY AG
Basel (Schweiz)

4-14417/1+2/-

Aminoniederalkyl-penem-Verbindungen, Verfahren zu ihrer Herstellung, pharmazeutische Präparate, welche diese Verbindungen enthalten, und Verwendung von letzteren

Die vorliegende Erfindung betrifft neue 2-Aminoniederalkyl-penem-Verbindungen, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere 2-Aminoniederalkyl-2-penem-Verbindungen der Formel

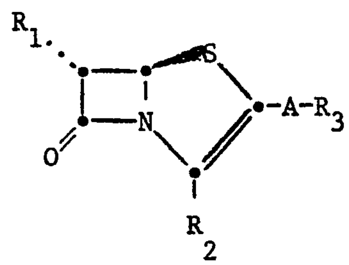 (I),

worin $R_1$ durch Hydroxy oder geschütztes Hydroxy substituiertes Niederalkyl ist, $R_2$ Carboxyl oder geschütztes Carboxyl $R_2'$ darstellt, $R_3$ Amino, durch Niederalkyl substituiertes Amino, substituiertes Methylenamino oder geschütztes Amino bedeutet und A durch Niederalkyl substituiertes geradkettiges Niederalkylen ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt, Salze von solchen Verbindungen der Formel I, die eine salzbildende Gruppe aufweisen, optische Isomere von Verbindungen

der Formel I und Mischungen dieser optischen Isomere, Verfahren zur Herstellung von Verbindungen der Formel I, pharmazeutische Präparate enthaltend solche Verbindungen und ihre Verwendung zur Herstellung von pharmazeutischen Präparaten oder als pharmakologisch wirksame Verbindungen.

Die vor- und nachstehend verwendeten Definitionen haben im Rahmen der vorliegenden Beschreibung vorzugsweise die folgenden Bedeutungen:

Durch Niederalkyl substituiertes Amino $R_3$ ist z.B. Niederalkylamino oder Diniederalkylamino.

In substituiertem Methylenamino ist der Methylenrest vorzugsweise mono- oder disubstituiert. Substituiertes Methylenamino ist z.B. eine Gruppe der Formel

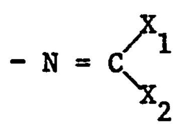

(IA)

worin $X_1$ Wasserstoff, gegebenenfalls substituiertes Amino, z.B. Amino, Niederalkylamino, Diniederalkylamino, Niederalkylenamino, Nitroamino, Hydrazino oder Anilino, veräthertes Hydroxy, z.B. Niederalkoxy oder Phenylniederalkoxy, veräthertes Mercapto, z.B. Niederalkylthio, gegebenenfalls substituiertes Niederalkyl, z.B. Niederalkyl, Aminoniederalkyl, N-Niederalkylaminoniederalkyl oder N,N-Diniederalkylaminoniederalkyl, Niederalkenyl, Phenyl oder monocyclisches Heteroaryl, wie entsprechendes 5- oder 6-gliedriges Heteroaryl mit 1 bis 2 Stickstoffatomen und/oder einem Sauerstoff- oder Schwefelatom, wie Pyridyl, z.B. 2- oder 4-Pyridyl, Thienyl, z.B. 2-Thienyl,oder Thiazolyl, z.B. 4-Thiazolyl ist, und $X_2$ gegebenenfalls substituiertes Amino, z.B. Amino, Niederalkylamino, Diniederalkylamino, Niederalkylenamino, Hydrazino oder Anilino, veräthertes Hydroxy, z.B. Niederalkoxy oder Phenylniederalkoxy, oder veräthertes Mercapto, z.B. Niederalkylthio, darstellt.

In bevorzugten Resten der Formel (IA) ist $X_1$ Wasserstoff, Amino, Niederalkylamino oder Niederalkyl und $X_2$ Amino.

Reste der Formel (IA), welche am α-Atom des Substituenten $X_1$ oder des Substituenten $X_2$ ein Wasserstoffatom besitzen, z.B. Reste der Formel (IA), worin $X_1$ Amino, Niederalkylamino, Nitroamino, Hydrazino, Anilino oder gegebenenfalls substituiertes Niederalkyl ist und/oder $X_2$ Amino, Niederalkylamino, Hydrazino oder Anilino ist, können auch in den tautomeren Formen

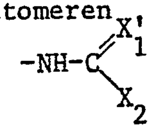

(IB)

oder

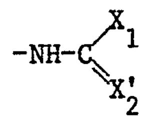

(IC) ,

worin $X_1'$ bzw. $X_2'$ entsprechendes substituiertes oder unsubstituiertes Methylen oder Imino ist, vorliegen.

In der vorliegenden Beschreibung bedeutet der im Zusammenhang mit Definitionen von Gruppen und Verbindungen verwendete Ausdruck "Nieder" das die entsprechenden Gruppen bzw. Verbindungen, sofern nicht ausdrücklich anders definiert, bis zu 7, bevorzugt bis zu 4 Kohlenstoffatome enthalten.

Durch Hydroxy substituiertes Niederalkyl $R_1$ ist insbesondere in α-Stellung zum Penem-Ringgerüst durch Hydroxy substituiertes Niederalkyl und bedeutet z.B. 1-Hydroxyprop-1-yl, 2-Hydroxyprop-2-yl, 1-Hydroxybut-1-yl, 2-Hydroxybut-2-yl und in erster Linie Hydroxymethyl oder 1-Hydroxyäthyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Di-n-butylamino bedeutet.

Niederalkylenamino weist insbesondere 4 bis 6 Kohlenstoffkettenglieder auf und bedeutet z.B. Pyrrolidino oder Piperidino.

Niederalkoxy ist z.B. Methoxy, Aethoxy, n-Propoxy, Isopropoxy, n-Butoxy oder tert.-Butoxy, während Phenylniederalkoxy z.B. Benzyloxy ist.

Niederalkylthio ist z.B. Methylthio, Aethylthio, n-Propylthio, Isopropylthio oder n-Butylthio.

Niederalkyl ist z.B. Methyl, Aethyl, n-Propyl, Isopropyl, n-Butyl, Isobutyl, sek.-Butyl oder tert.-Butyl.

Aminoniederalkyl ist z.B. 2-Aminoäthyl oder 3-Aminopropyl.

N-Niederalkylaminoniederalkyl ist z.B. 2-Methyl- oder 2-Aethylaminoäthyl, während N,N-Diniederalkylaminoniederalkyl z.B. 2-Dimethylaminoäthyl oder 2-Diäthylaminoäthyl bedeutet.
Niederalkenyl ist z.B. Allyl, n-Propenyl oder Isopropenyl.

Geradkettiges Niederalkylen im Rest A weist 1 bis 7, vorzugsweise 1 bis 4 Kohlenstoffatome auf und ist z.B. Methylen, Aethylen, 1,3-Propylen, 1,4-Butylen, ferner auch 1,5-Pentylen. Ein solcher Niederalkylenrest ist durch Niederalkyl mit 1 bis 4 Kohlenstoffatomen, insbesondere mit 1 bis 2 Kohlenstoffatomen, wie n-Propyl, n-Butyl oder insbesondere Methyl oder Aethyl, mono- oder polysubstituiert, wie insbesondere mono- oder disubstituiert. Zwei Niederalkylsubstituenten können am gleichen Kohlenstoffatom (geminal), an benachbarten Kohlenstoffatomen (vicinal) oder an zwei verschiedenen, durch mindestens eine Methylengruppe getrennten Kohlenstoffatomen der Niederalkylen-Kette angeordnet sein.

Ein solcher Rest A ist beispielsweise Niederalkyliden, wie Aethyliden ("Methyl-methylen"), 1,2-Propylen, 2-Niederalkyl-, wie 2-Methyl-1,2-propylen, 1,2-Butylen, 2-Niederalkyl-, wie 2-Methyl-

oder 2-Aethyl-1,2-butylen, 3-Niederalkyl-, wie 3-Methyl-1,2-butylen, 1,3-Butylen, 2-Niederalkyl-, wie 2-Methyl-1,3-butylen, 3-Niederalkyl-, wie 3-Methyl-1,3-butylen, oder 2,2-Diniederalkyl-, wie 2,2-Dimethyl-1,3-butylen, wobei in diesen Resten das Kohlenstoffatom 1 mit dem Penem-Ringgerüst verbunden ist, oder auch 1,2-Propylen, 2-Niederalkyl-, wie 2-Methyl-1,2-propylen, 1,2-Butylen, 2-Niederalkyl-, wie 2-Methyl- oder 2-Aethyl-1,2-butylen, 3-Niederalkyl-, wie 3-Methyl-1,2-butylen, oder 3,3-Diniederalkyl-, wie 3,3-Dimethyl-1,2-butylen, wobei in diesen Resten das Kohlenstoffatom 2 mit dem Penem-Ringgerüst verbunden ist. Bevorzugte Reste A sind über das Kohlenstoffatom 1 mit dem Penem-Ringgerüst verbundenes 1,2-Propylen, 1,2-Butylen, 1,3-Butylen und 2-Methyl-1,2-propylen, sowie über das Kohlenstoffatom 2 mit dem Penem-Ringgerüst verbundenes 1,2-Propylen.

Die in den Verbindungen der Formel I vorhandenen funktionellen Gruppen, wie Hydroxy-, Carboxy- oder Aminogruppen, insbesondere die Hydroxygruppe im Rest $R_1$, die Carboxylgruppe $R_2$ und eine Aminogruppe $R_3$, sind gegebenenfalls durch konventionelle Schutzgruppen geschützt, die in den Penem-, Penicillin-, Cephalosporin- und Peptidchemie verwendet werden.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in

J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und

Houben-Weyl, "Methoden der Organischen Chemie", Band 15/1, Georg Thieme Verlag, Stuttgart, 1974.

In Verbindungen der Formel (I) kann eine Hydroxygruppe im Rest $R_1$ beispielsweise durch Acylreste geschützt sein. Geeignete Acylreste sind z.B. gegebenenfalls durch Halogen substituiertes Niederalkanoyl, z.B. Acetyl- oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, oder gegebenenfalls durch Nitro substituiertes Phenylniederalkoxycarbonyl, z.B. 4-Nitrobenzyloxycarbonyl. Weitere geeignete Hydroxyschutzgruppen sind z.B. trisubstituiertes Silyl, wie Triniederalkylsilyl, z.B. Trimethylsilyl oder tert.-Butyl-dimethylsilyl, 2-Halogenniederalkylgruppen, z.B. 2-Chlor-, 2-Brom-, 2-Jod- und 2,2,2-Trichloräthyl, und gegebenenfalls durch Halogen, z.B. Chlor, Niederalkoxy, z.B. Methoxy, und/oder Nitro substituiertes Phenylniederalkyl, wie entsprechendes Benzyl. Bevorzugt als Hydroxyschutzgruppe ist die Triniederalkylsilyl-Gruppe.

Eine Carboxylgruppe $R_2$ ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist. Eine geschützte Carboxylgruppe $R_2'$ kann insbesondere auch eine unter physiologischen Bedingungen spaltbare oder leicht in eine andere funktionell abgewandelte Carboxylgruppe, wie in eine andere veresterte Carboxylgruppe umwandelbare, veresterte Carboxylgruppe darstellen.

Solche veresterten Carboxylgruppen $R_2'$ enthalten als veresternde Gruppen in erster Linie in 1-Stellung verzweigte oder in 1- oder 2-Stellung geeignet substituierte Niederalkylgruppen. Bevorzugte in veresterter

Form vorliegende Carboxylgruppen sind unter anderen Niederalkoxycarbonyl, z.B. Methoxycarbonyl, Aethoxycarbonyl, Isopropoxycarbonyl oder tert.-Butoxycarbonyl, und (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls, z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder $\omega$-Halogenniederalkoxycarbonyl, worin Niederalkoxy 4 bis 7 Kohlenstoffatome enthält, z.B. 4-Chlorbutoxycarbonyl, Phthalimidomethoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl.

Weitere in veresterter Form vorliegende geschützte Carboxylgruppen $R_2'$ sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Aethyl, ferner Niederalkoxy, z.B. Methoxy, als Substituenten. Geeignete Silyl- bzw. Stannylgruppen sind in erster Linie Triniederalkylsilyl, insbesondere Trimethylsilyl oder Dimethyl-tert.-butyl-silyl, oder entsprechend substituierte Stannylgruppen, z.B. Tri-n-butylstannyl.

Eine unter physiologischen Bedingungen spaltbare veresterte Carboxylgruppe $R_2'$ ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet, 1-Niederalkoxyniederalkoxycarbonyl oder auch 1-Niederalkoxycarbonyloxy-niederalkoxycarbonyl, worin Niederalkyl z.B. Methyl, Propyl, Butyl oder insbesondere Aethyl und Niederalkoxy z.B. Methoxy, Propoxy oder Butoxy ist. Solche Gruppen sind z.B. Niederalkanoyloxymethoxycarbonyl, z.B. Acetoxymethoxycarbonyl oder Pivaloyloxymethoxycarbonyl, Aminoniederalkanoyloxymethoxycarbonyl, insbesondere α-Aminoniederalkanoyloxymethoxycarbonyl, z.B. Glycyloxymethoxycarbonyl, L-Valyloxymethoxycarbonyl, L-Leucyloxymethoxycarbonyl, Phthalidyloxycarbonyl, 4-Crotonolactonyl oder 4-Butyrolacton-4-yl, Indanyloxycarbonyl, z.B. 5-Indanyloxycarbonyl, 1-Aethoxycarbonyloxyäthoxycarbonyl, Methoxymethoxycarbonyl oder 1-Methoxyäthoxycarbonyl.

Bevorzugte geschützte Carboxylgruppen $R_3'$ sind die 4-Nitrobenzyloxycarbonyl-, Niederalkenyloxycarbonyl- und die in 2-Stellung durch Niederalkylsulfonyl, Cyano oder Triniederalkylsilyl substituierte Aethoxycarbonylgruppe, sowie vor allem die unter physiologischen Bedingungen spaltbaren veresterten Carboxylgruppen, wie z.B. Niederalkanoyloxymethoxycarbonyl oder 1-Niederalkoxycarbonyloxy-niederalkoxycarbonyl.

Eine geschützte Aminogruppe kann beispielsweise in Form einer leicht spaltbaren Acylamino-, Acylimino-, verätherten Mercaptoamino-, Silyl- oder Stannylaminogruppe oder als Enamino-, Nitro- oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer gegebenenfalls, z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1- oder 2-Stellung substituiertes Niederalkoxycarbonyl geeignet, wie Niederalkoxycarbonyl, z.B. Methoxy- oder Aethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl, oder 2-Triarylsilyläthoxycarbonyl, wie 2-Triphenylsilyläthoxycarbonyl.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält. Entsprechende Silyl- oder Stannylgruppen sind in erster Linie Triniederalkylsilyl, insbesondere Trimethylsilyl, ferner Dimethyl-tert.-butylsilyl, oder entsprechend substituiertes Stannyl, z.B. Tri-n-butylstannyl.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Schutzgruppen dieser Art sind beispielsweise 1-Acyl-niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -äthylhalbesters, und Niederalk-1-en insbesondere 1-Propen bedeutet. Entsprechende Schutzgruppen sind in erster Linie 1-Niederalkanoylprop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Bevorzugte geschützte Aminogruppen sind z.B. Azido, Phthalimido, Nitro, Niederalkenyloxycarbonylamino, gegebenenfalls durch Nitro substituiertes Benzyloxycarbonylamino, 1-Niederalkanoyl-niederalk-1-en-2-ylamino oder 1-Niederalkoxycarbonyl-niederalk-1-en-2-ylamino.

Salze von erfindungsgemässen Verbindungen sind in erster Linie pharmazeutisch annehmbare, nichttoxische Salze von Verbindungen der Formel I. Solche Salze werden beispielsweise von Verbindungen der Formel I gebildet, worin $R_2$ Carboxyl ist und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethylpiperidin, Cycloalkylaminen, z.B. Dicyclohexylamin, oder Benzylaminen, z.B. N,N'-Dibenzyläthylendiamin, Dibenzylamin oder N-Benzyl-β-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salzen, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penemverbindungen der Formel I weisen am Kohlenstoffatom 5 die R-Konfiguration und am Kohlenstoffatom 6 die S-Konfiguration auf.

Die Verbindungen der Formel I können in den Substituenten $R_1$ und/oder A weitere Chiralitätszentren besitzen, welche jeweils in der R-, S- oder der racemischen R,S-Konfiguration vorliegen können. In Verbindungen der Formel I, worin $R_1$ in α-Stellung (am Kohlenstoffatom 1') durch Hydroxy asymmetrisch substituiertes Niederalkyl mit 2 bis 7 Kohlenstoffatomen, insbesondere 1'-Hydroxyäthyl, ist, nehmen die Substituenten am Kohlenstoffatom 1' vorzugsweise die R-Konfiguration ein.

Die Erfindung betrifft insbesondere Verbindungen der Formel I, worin $R_1$ durch Hydroxy oder geschütztes Hydroxy substituiertes Niederalkyl ist, $R_2$ Carboxyl oder geschütztes Carboxyl $R_2'$ bedeutet, $R_3$ Amino, Niederalkylamino, Diniederalkylamino, eine Gruppe der Formel $-N=C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl, Pyridyl, z.B. 2- oder 4-Pyridyl, Thienyl, z.B. 2-Thienyl, oder Thiazolyl, z.B. 4-Thiazolyl ist, und $X_2$ Amino, Niederalkylamino, Diniederalkylamino, Niederalkylenamino, Hydrazino, Anilino, Niederalkoxy, Phenylniederalkoxy oder Niederalkylthio bedeutet, oder geschütztes Amino ist, und A durch Niederalkyl mit 1 bis 4 Kohlenstoffatomen substituiertes geradkettiges Niederalkylen darstellt, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt; Salze von solchen Verbindungen der Formel I, die eine salzbildende Gruppe enthalten, optische Isomere von Verbindungen der Formel I, welche in den Resten $R_1$ und/oder A Chiralitätszentren besitzen, und Mischungen dieser optischen Isomere.

Die Erfindung betrifft vor allem Verbindungen der Formel I, worin $R_1$ durch Hydroxy, Triniederalkylsilyloxy, 2-Halogenniederalkoxy,

2-Halogenniederalkoxycarbonyloxy oder gegebenenfalls durch Nitro substituiertes Phenylniederalkoxycarbonyloxy substituiertes Niederalkyl ist, $R_2$ Carboxyl, Niederalkenyloxycarbonyl, gegebenenfalls durch Nitro substituiertes Benzyloxycarbonyl, Niederalkanoylmethoxycarbonyl, 2-Halogenniederalkoxycarbonyl, 2-Triniederalkylsilyläthoxycarbonyl oder eine unter physiologischen Bedingungen spaltbare veresterte Carboxylgruppe, z.B. 1-Niederalkoxycarbonyloxyniederalkoxycarbonyl, Niederalkanoyloxymethoxycarbonyl, α-Aminoniederalkanoyloxymethoxycarbonyl oder Phthalidyloxycarbonyl, bedeutet, $R_3$ Amino, Niederalkylamino, Diniederalkylamino, eine Gruppe der Formel $-N=C(X_1,X_2)$, worin $X_1$ Wasserstoff, Amino, Niederalkylamino, Niederalkyl, Phenyl oder Pyridyl, z.B. 2-Pyridyl, und $X_2$ Amino, Niederalkylamino oder Diniederalkylamino ist; Azido, Phthalimino, Nitro, Niederalkenyloxycarbonylamino, gegebenenfalls durch Nitro substituiertes Benzyloxycarbonylamino, 1-Niederalkanoyl-niederalk-1-en-2-ylamino oder 1-Niederalkoxycarbonyl-niederalk-1-en-2-ylamino bedeutet, und A durch Niederalkyl mit 1 bis 4 Kohlenstoffatomen mono- oder disubstituiertes geradkettiges Niederalkylen darstellt, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt; Salze, von solchen Verbindungen der Formel I, die eine salzbildende Gruppe aufweisen, optische Isomere von Verbindungen der Formel I, welche in den Resten $R_1$ und/oder A Chiralitätszentren besitzen, und Mischungen dieser optischen Isomere.

Die Erfindung betrifft in erster Linie Verbindungen der Formel I, worin $R_1$ durch Hydroxy substituiertes Niederalkyl und A durch Methyl oder Aethyl monosubstituiertes geradkettiges Niederalkylen mit 1 bis 4 Kohlenstoffatomen bedeutet oder worin $R_1$ Hydroxymethyl ist und A durch Methyl oder Aethyl disubstituiertes geradkettiges Niederalkylen mit 1 bis 4 Kohlenstoffatomen bedeutet, und worin

$R_2$ jeweils Carboxyl, 1-Niederalkoxycarbonyloxyniederalkoxycarbonyl oder Niederalkanoyloxymethoxycarbonyl und $R_3$ jeweils Amino, Niederalkylamino oder Formamidino darstellen, Salze von solchen Verbindungen der Formel I, optische Isomere von Verbindungen der Formel I, welche in den Resten $R_1$ und/oder A Chiralitätszentren besitzen, und Mischungen dieser optischen Isomere.

Die Erfindung betrifft hauptsächlich Verbindungen der Formel I, worin $R_1$ Hydroxymethyl oder 1-Hydroxyäthyl bedeutet und A durch Methyl oder Aethyl monosubstituiertes Aethylen oder 1,3-Propylen darstellt oder worin $R_1$ Hydroxymethyl ist und A durch Methyl disubstituiertes Aethylen bedeutet, und worin $R_2$ jeweils Carboxyl und $R_3$ jeweils Amino darstellen, und Salze von solchen Verbindungen der Formel I.

Die Erfindung betrifft weiterhin die reinen optischen Isomere von solchen Verbindungen der Formel I, welche in den Substituenten $R_1$ und/oder A weitere Chiralitätszentren besitzen, insbesondere das (1'R)-Isomere von Verbindungen der Formel I, worin $R_1$ 1'-Hydroxyäthyl ist, und Salze von solchen Verbindungen der Formel I.

Die Erfindung betrifft insbesondere die in den Beispielen genannten Verbindungen der Formel I und ihre Salze.

Die Verbindungen der vorliegenden Erfindung können nach an sich bekannten Verfahren hergestellt werden.

Die neuen Verbindungen werden z.B. hergestellt, indem man

a) eine Ylid-Verbindung der Formel

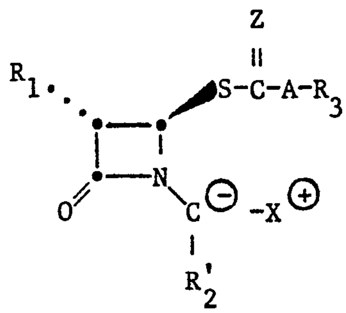

(II),

worin $R_1, R_2'$, $R_3$ und A die unter Formel I angegebenen Bedeutungen haben, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

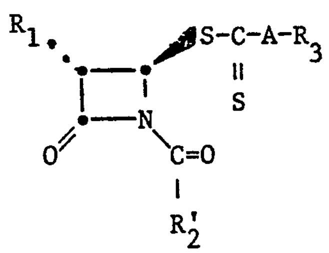

(III),

worin $R_1$, $R_2'$, $R_3$ und A die unter Formel I angegebenen Bedeutungen haben, mit einer organischen Verbindung des dreiwertigen Phosphors behandelt, und wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_2'$ in die freie Carboxylgruppe oder in eine andere geschützte Carboxylgruppe $R_2'$ oder eine freie Carboxylgruppe $R_2$ in eine geschützte Carboxylgruppe $R_2'$ überführt, und/oder, wenn erwünscht, eine geschützte Aminogruppe $R_3$ in die freie Aminogruppe oder eine freie Aminogruppe $R_3$ in eine substituierte Aminogruppe überführt, und/oder, wenn erwünscht, eine erhältliche Verbindung mit salzbildender

Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

In den Ausgangsverbindungen der Formeln II und III sind funktionelle Gruppen, wie eine freie Hydroxygruppe im Rest $R_1$ und insbesondere eine freie Aminogruppe $R_3$, vorzugsweise durch konventionelle Schutzgruppen, z.B. durch eine der oben genannten, geschützt.

a) Cyclisierung der Verbindung der Formel II

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetall-, z.B. Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. Natriumion. .

Die Ylid-Verbindungen der Formel II werden in der isomeren Ylen-Form auch als Phosphoran-Verbindungen bezeichnet. In Phosphonio-Verbindungen der Formel II wird die negative Ladung durch die positiv geladene Phosphoniogruppe neutralisiert. In Phosphono-Verbindungen der Formel II wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30° C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Hexan oder Benzol, einem halogenierten Kohlenwasserstoff, z.B. Methylenchlorid, einem Aether, z.B. Diäthyläther, einem Carbonsäureamid, z.B. Dimethylformamid, einem Diniederalkylsulfoxid, z.B. Dimethylsulfoxid, oder einem Niederalkanol, z.B. Methanol, oder in einem Gemisch davon, und, falls notwendig, in einer Inertgas-, z.B. Stickstoffatmosphäre, durchgeführt.

b. Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2-N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Trialkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis etwa 80°C, bevorzugt von etwa 40° bis etwa 60°C, durchgeführt, wobei man 1 Moläquivalent einer Verbindung der Formel III mit 2 Moläquivalenten der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel III in situ, wie weiter unten unter Stufe 1.5 beschrieben, her und setzt es ohne Isolierung aus dem Reaktionsgemisch mit der organischen Verbindung des dreiwertigen Phosphors um, wobei die Endprodukte der Formel I entstehen.

Bevorzugt werden solche Ausgangsmaterialien der Formeln II und III verwendet, die zu den eingangs als besonders bevorzugt genannten Verbindung der Formel I führen.

In einer erhältlichen Verbindung der Formel I, worin eine oder mehrere funktionelle Gruppen geschützt sind, können diese, z.B. geschützte Amino-, Carboxyl- oder Hydroxygruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer erfindungsgemäss erhältlichen Verbindung der Formel I mit einer geschützten Aminogruppe $R_3$ kann diese in an sich bekannter Weise, z.B. je nach Art der Schutzgruppe, vorzugsweise mittels Solvolyse oder Reduktion, in die freie Aminogruppe $R_3$ übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie

Zink in Gegenwart einer geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino, durch Umsetzen mit einer Verbindung des Palladiums, z.B. Tetrakis(triphenylphosphin)palladium, in Gegenwart von Triphenylphosphin und Behandeln mit einer Carbonsäure, z.B. 2-Aethylhexansäure, oder einem Salz davon, gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanionen liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid oder Palladium oder durch Behandeln mit Zink in Gegenwart einer Säure, wie Essigsäure. Eine in Form einer Phthalimidogruppe geschützte Aminogruppe

kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

Ferner kann man eine freie Aminogruppe $R_3$ in an sich bekannter Weise in eine substituierte Aminogruppe überführen. So kann man beispielsweise Amino durch Umsetzen mit einem entsprechenden Acylhalogenid, wie -chlorid, in Acylamino $R_3$ und mit einer β-Dicarbonylverbindung, wie einem 1-Niederalkanoyl-aceton oder einem Acetessigsäure-niederalkylester in 1-Niederalkanoyl- bzw. 1-Niederalkoxycarbonylprop-1-en-2-ylamino überführen. Die Umwandlung von Aminogruppen in Amidino-, Guanidino-, Isoharnstoff-, Isothioharnstoff-, Imidoäther- und Imidothioäthergruppen kann beispielsweise nach einem der in der Deutschen Offenlegungsschrift Nr. 26 52 679 genannten Verfahren durchgeführt werden. So können beispielsweise Verbindungen der Formel I, worin $R_3$ Amino darstellt, durch Umsetzen mit Trimethylsilylchlorid und einem Imidohalogenid der Formel $[(X_1,Y_1)C{=}X_2]^{\oplus}Y_2^{\ominus}$, worin $Y_1$ Halogen, z.B. Chlor, und $Y_2$ ein Anion, z.B. Chlorid, bedeutet, in Amidine und durch Umsetzen mit einem substituierten Isoharnstoff bzw. Isothioharnstoff der Formel $(X_1Y_3)C{=}X_2$, worin $Y_3$ Niederalkoxy oder Niederalkylthio ist, in Guanidine übergeführt werden. Weiterhin kann man eine freie Aminogruppe $R_3$ in eine durch Niederalkyl mono- oder disubstituierte Aminogruppe überführen. Die Einführung der Niederalkylgruppe(n) erfolgt beispielsweise durch Umsetzung mit entsprechenden reaktionsfähigen Niederalkylestern, wie -halogeniden, z.B. -chloriden oder -bromiden, oder -sulfonaten, z.B. -methan- oder -p-toluolsulfonaten, in Gegenwart eines basischen Kondensationsmittels, wie eines Alkali- oder Erdalkalimetallhydroxids oder -carbonats, z.B. Kaliumhydroxid oder Natriumcarbonat, in einem inerten Lösungsmittel, wie einem Niederalkanol, bei Raumtemperatur oder erhöhter oder erniedrigter Temperatur, z.B. bei etwa -20° bis +80°C.

In einer verfahrensgemäss erhältlichen Verbindung der Formel I, worin $R_2$ eine geschützte Carboxylgruppe $R_2'$ bedeutet, kann die Carboxylgruppe in an sich bekannter Weise freigesetzt werden.

So kann man tert.-Niederalkoxycarbonyl oder in 2-Stellung durch eine trisubstituierte Silylgruppe oder in 1-Stellung durch Niederalkoxy substituiertes Niederalkoxycarbonyl oder gegebenenfalls substituiertes Diphenylmethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzen mit einer Verbindung des Palladiums, z.B. Tetrakis(triphenylphosphin)palladium, in Gegenwart von Triphenylphosphin und unter Zusatz einer Carbonsäure, z.B. 2-Aethylhexansäure, oder einem Salz davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagenz, wie Natriumthiophenolat oder Natriumjodid gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

In verfahrensgemäss erhältlichen Verbindungen der Formel I, worin der Rest $R_1$ durch geschütztes Hydroxy substituiert ist, kann die geschützte Hydroxygruppe in an sich bekannter Weise in die freie Hydroxygruppe überführt werden. Beispielsweise wird eine durch eine geeignete Acylgruppe oder eine organische Silyl- oder Stannylgruppe geschützte Hydroxygruppe wie eine entsprechend geschützte Aminogruppe freigesetzt, eine Triniederalkylsilylgruppe z.B. mit Tetrabutylammoniumfluorid und Essigsäure (Unter diesen Bedingungen werden durch trisubstituierte Silyläthylgruppen geschützte Carboxylgruppen nicht gespalten). Eine 2-Halogenniederalkylgruppe und eine gegebenenfalls substituierte Benzylgruppe werden reduktiv abgespalten.

Andererseits können auch Verbindungen der Formel I, worin $R_2$ Carboxy, bedeutet, in Verbindungen der Formel I überführt werden, worin $R_2$ eine geschützte Carboxylgruppe $R_2'$, insbesondere eine veresterte Carboxyl-

gruppe $R_2'$, und in erster Linie eine unter physiologischen Bedingungen spaltbare veresterte Carboxylgruppe, darstellt. So kann man die freie Carboxylgruppe z.B. durch Behandeln mit einer geeigneten Diazoverbindung, wie einem Diazoniederalkan, z.B. Diazomethan, oder einem Phenyldiazoniederalkan, z.B. Diphenyldiazomethan, wenn notwendig in Gegenwart einer Lewis-Säure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodiimid, sowie Carbonyldiimidazol, verestern. Ester können auch durch Umsetzung eines gegebenenfalls in situ hergestellten Salzes der Säure mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylestern, wie Chlorameisensäureäthyl- oder Chlorameisensäureisobutylester, oder mit Halogenessigsäurehalogeniden, wie Trichloressigsäurechlorid) durch Umsetzen mit Alkoholen, gegebenenfalls in Gegenwart einer Base, wie Pyridin, in eine veresterte Carboxylgruppe übergeführt werden.

In einer Verbindung der Formel I mit einer veresterten Carboxylgruppe $R_2'$ kann diese in eine andere veresterte Carboxylgruppe $R_2'$ überführt werden, z.B. 2-Chloräthoxycarbonyl oder 2-Bromäthoxycarbonyl durch Behandeln mit einem Jodsalz, z.B. Natriumjodid, in 2-Jodäthoxycarbonyl. Ferner kann man in Verbindungen der Formel I, die eine in veresterter Form geschützte Carboxylgruppe $R_2'$ enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähigen Ester eines entsprechenden Alkohols in eine Verbindung der Formel I überführen, worin $R_2$ eine unter physiologischen Bedingungen spaltbare veresterte Carboxylgruppe darstellt.

Salze von Verbindungen der Formel I mit salzbildenden Gruppen können in an sich bekannter Weise hergestellt werden. So kann man Salze von Verbindungen der Formel I mit einer freien Carboxylgruppe $R_2$ z.B. durch Behandeln mit Metallverbindungen, wie Alkalimetallsalzen von geeigneten organischen Carbonsäuren, z.B. dem Natriumsalz der $\alpha$-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von Isomeren können nach an sich bekannten Methoden in die einzelnen Isomeren aufgetrennt werden, Gemische von diastereomeren Isomeren z.B. durch fraktioniertes Kristallisieren, Adsorptionschromatographie (Kolonnen- oder Dünnschichtchromatographie) oder andere geeignete Trennverfahren.

Die Spaltung von erhaltenen Racematen in ihre optischen Antipoden kann auf verschiedenen Wegen erfolgen.

Einer dieser Wege besteht darin, dass man ein Racemat mit einem optisch aktiven Hilfsstoff reagieren lässt, das dabei entstandene

Gemisch zweier diastereomerer Verbindungen mit Hilfe von geeigneten physikalisch-chemischen Methoden trennt und die einzelnen diastereomeren Verbindungen dann in die optisch aktiven Verbindungen spaltet.

Zur Trennung in Antipoden besonders geeignete Racemate sind solche, die eine saure Gruppe besitzen, wie z.B. Racemate von Verbindungen der Formel I, worin $R_2$ Carboxy ist. Diese sauren Racemate können mit optisch aktiven Basen, z.B. Estern von optisch aktiven Aminosäuren, oder (-)-Brucin, (+)-Chinidin, (-)-Chinin, (+)-Cinchonin, (+)-Dehydroabietylamin, (+)- und (-)-Ephedrin, (+)- und (-)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol, (+)- oder (-)-2-Octanol verestert sein oder werden, worauf nach erfolgter Isolierung des gewünschten Diastereomeren die Carboxylgruppe freigesetzt wird.

Zur Racemattrennung kann auch die Hydroxygruppe mit optisch aktiven Säuren oder deren reaktionsfähigen, funktionellen Derivaten verestert werden, wobei sich diastereomere Ester bilden. Solche Säuren sind beispielsweise (-)-Abietinsäure, D(+)- und L(-)-Aepfelsäure, N-acylierte optisch aktive Aminosäure, (+)- und (-)-Camphansäure, (+)- und (-)-Ketopinsäure, L(+)-Ascorbinsäure, (+)-Camphersäure, (+)-Campher-10-sulfonsäure(β), (+)- oder (-)-α-Bromcampher-π-sulfonsäure, D(-)-Chinasäure, D(-)-Isoascorbinsäure, D(-)- und L(+)-Mandelsäure, (+)-1-Menthoxyessigsäure, D(-)- und L(+)-Weinsäure und deren Di-O-benzoyl- und Di-O-p-toluylderivate.

Durch Umsetzung mit optisch aktiven Isocyanaten, wie mit (+)- oder (-)-1-Phenyläthylisocyanat, können Verbindungen der Formel (I), worin

$R_2$ geschütztes Carboxy und $R_1$ durch Hydroxy substituiertes Niederalkyl bedeutet, in ein Gemisch diastereomerer Urethane umgewandelt werden.

Basische Racemate, z.B. Verbindungen der Formel I, worin der Rest $R_3$ eine Aminogruppe ist, können mit den genannten optischen aktiven Säuren diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch aktiven Absorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden gegenüber biologischem Material wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produktes aus.

Die Auftrennung von Diastereomeren in die einzelnen Racemate und der Racemate in die optischen Antipoden, kann auf einer beliebigen Verfahrensstufe, d.h. z.B. auch auf der Stufe der Ausgangsverbindungen der Formeln II oder III oder auf einer beliebigen Stufe des nachstehend beschriebenen Verfahrens zur Herstellung der Ausgangsverbindungen der Formel II, durchgeführt werden.

Bei allen nachträglichen Umwandlungen erhaltener Verbindungen der Formel I werden solche Reaktionen bevorzugt, die unter neutralen oder alkalischen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, gebildet werden. Beispielsweise kann ein Ausgangsmaterial der Formel II, worin Z Sauerstoff ist, aus einer Verbindung der Formel II, worin Z eine, wie nachstehend beschrieben, gegebenenfalls substituierte Methylidengruppe ist, durch Ozonisierung und anschliessende Reduktion des gebildeten Ozonids, analog dem weiter unten angegebenen Verfahren (Stufe 2.3), in situ hergestellt werden, worauf in der Reaktionslösung die Cyclisierung zur Verbindung der Formel I erfolgt.

Die Ausgangsverbindungen der Formeln II und III und die Vorstufen können, wie in den Reaktionsschemata I und II angegeben, hergestellt werden:

Reaktionsschema I

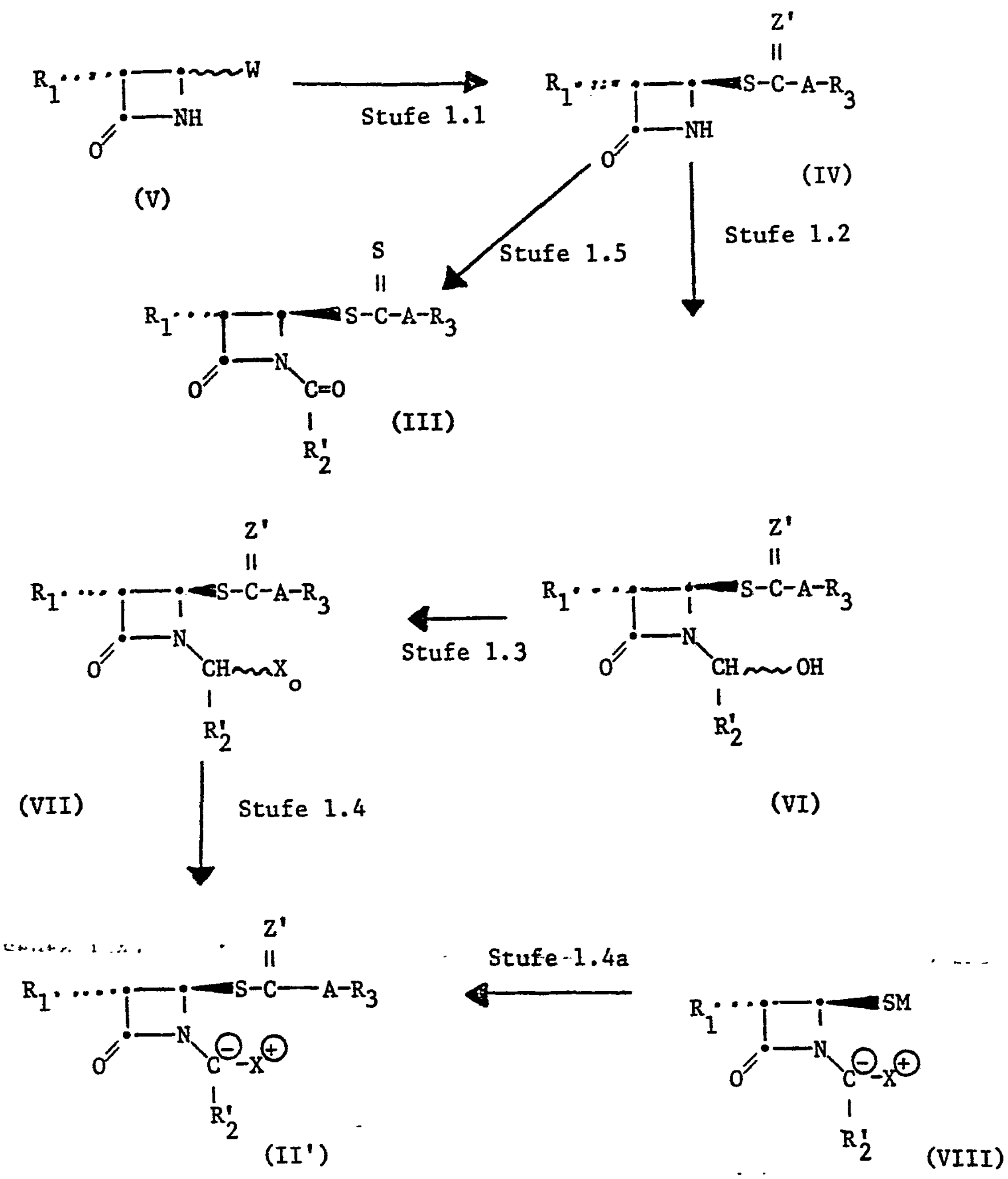


In den Verbindungen der Formeln IV, VI, VII und II' ist Z' Sauerstoff, Schwefel oder auch eine gegebenenfalls durch einen oder zwei Substituenten Y substituierte Methylidengruppe, die durch Oxidation in eine Oxogruppe Z überführt werden kann. Ein Substituent Y

dieser Methylidengruppe ist ein organischer Rest, beispielsweise gegebenenfalls substituiertes Niederalkyl, z.B. Methyl oder Aethyl, Cycloalkyl, z.B. Cyclopentyl oder Cyclohexyl, Phenyl oder Phenylniederalkyl, z.B. Benzyl, oder insbesondere eine, inklusive mit einem optisch aktiven Alkohol, wie 1-Menthol, veresterte Carboxylgruppe, z.B. einer der unter $R_2$ erwähnten gegebenenfalls substituierten Niederalkoxycarbonyl- oder Arylmethoxycarbonylreste oder auch 1-Menthyloxycarbonyl. Die Methylidengruppen Z' trägt bevorzugt einen der genannten Substituenten. Hervorzuheben sind die Methoxycarbonylmethyliden- und die Aethoxycarbonylmethylidengruppe.

In den Verbindungen der Formeln IV bis VIII sowie II' enthält der Rest $R_1$ bevorzugt eine der genannten geschützten Hydroxygruppen, z.B. gegebenenfalls substituiertes 1-Phenylniederalkoxy, gegebenenfalls substituiertes Phenylniederalkoxycarbonyloxy, oder trisubstituiertes Silyloxy, und eine freie Aminogruppe $R_3$ liegt bevorzugt in geschützter Form vor.

<u>Stufe 1.1:</u> Ein Thio-azetidinon der Formel IV wird erhalten, indem man ein 4-W-Azetidinon der Formel V, worin W eine nucleofuge Abgangsgruppe bedeutet, mit einer Mercaptoverbindung der Formel

$$R_3\text{-A-C(=Z')-SH} \qquad \text{(IX)}$$

oder einem Salz, z.B. einem Alkalimetall-, wie Natrium- oder Kaliumsalz davon, behandelt, und, wenn erwünscht, in einer erhältlichen Verbindung der Formel IV, worin $R_1$ durch Hydroxy substituiertes Niederalkyl ist, Hydroxy in geschütztes Hydroxy überführt.

Die nucleofuge Abgangsgruppe W in einem Ausgangsmaterial der Formel V ist ein durch den nucleophilen Rest

$$R_3\text{-A-C(=Z')-S-}$$

ersetzbarer Rest. Solche Gruppen W sind beispielsweise Acyloxyreste, Sulfonylreste $R_o\text{-}SO_2\text{-}$, worin $R_o$ ein organischer

Rest ist, Azido oder Halogen. In einem Acyloxyrest W ist Acyl z.B. der Rest einer organischen Carbonsäure, inklusive einer optisch aktiven Carbonsäure, und bedeutet beispielsweise Niederalkanoyl, z.B. Acetyl oder Propionyl, gegebenenfalls substituiertes Benzoyl, z.B. Benzoyl oder 2,4-Dinitrobenzoyl, Phenylniederalkanoyl, z.B. Phenylacetyl, oder den Acylrest einer der oben genannten optisch aktiven Säuren. In einem Sulfonylrest $R_o$-$SO_2$- ist $R_o$ beispielsweise gegebenenfalls durch Hydroxy substituiertes Niederalkyl, wie Methyl, Aethyl oder 2-Hydroxyäthyl, ferner auch entsprechendes substituiertes optisch aktives Niederalkyl, z.B. (2R)- oder (2S)-1-Hydroxyprop-2-yl, durch einen optisch aktiven Rest substituiertes Methyl, wie Campheryl, oder Benzyl, oder gegebenenfalls substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl- oder 2-Hydroxyäthylsulfonyl, Acetoxy oder Chlor.

Die nucleophile Substitution kann unter neutralen oder schwach basischen Bedingungen in Gegenwart von Wasser und gegebenenfalls einem mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, Acetonitril und ähnliche verwendet werden. Die Reaktion wird üblicherweise bei Raumtemperatur durchgeführt, kann aber auch bei erhöhter oder erniedrigter Temperatur durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

In die Reaktion können sowohl (3S,4S)- als auch (3S,4R)-konfigurierte Verbindungen der Formel V oder Mischungen davon eingesetzt werden. Die eintretende Gruppe $R_3$-A-C(=Z')-S- wird von der Gruppe $R_1$ bevorzugt in die trans-Stellung dirigiert, unabhängig davon, ob W zur Gruppe $R_1$ in cis- oder trans-Stellung steht. Obwohl die trans-(3S,4R)-Isomeren überwiegend gebildet werden, können doch gelegentlich auch die cis-Isomeren isoliert werden. Die Auftrennung der cis- und trans-Isomeren erfolgt wie oben beschrieben nach konventionellen Methoden, insbesondere durch Chromatographie und/oder durch Kristallisation.

Die nachträgliche Ozonisierung einer Methyliden-Gruppe Z' kann wie weiter unten angegeben durchgeführt werden. Ein erhaltenes Racemat der Formel IV kann in die optisch aktiven Verbindungen getrennt werden.

Ein Azetidinon der Formel V, worin $R_1$ Acetoxymethyl bedeutet, ist in der Deutschen Offenlegungsschrift Nr. 29 50 898 beschrieben. Andere Azetidinone der Formel V können nach an sich bekannten Verfahren hergestellt werden, beispielsweise indem man einen Vinylester der Formel $R_1$-CH=CH-W mit Chlorsulfonylisocyanat umsetzt und das entstandene Cycloaddukt mit einem Reduktionsmittel, z.B. Natriumsulfit, umsetzt. Bei dieser Synthese werden gewöhnlich Mischungen von cis- und trans-Isomeren erhalten, die in die reinen (3S)-Isomeren, z.B. durch Chromatographie und/oder Kristallisation oder Destillation, aufgetrennt werden können. Erhaltene racemische (3S,4RS)-Isomere können wie oben beschrieben in die reinen (3S,4R)-Antipoden getrennt werden. Die optisch aktiven Verbindungen der Formel V können auch nach dem unten in dem Reaktionsschema II angegebenen Verfahren hergestellt werden.

Stufe 1.2: Eine α-Hydroxycarbonsäureverbindung der Formel VI wird erhalten, indem man eine Verbindung der Formel IV mit einer Glyoxylsäure-Verbindung der Formel $OHC-R_2^{\prime}$ oder einem geeigneten Derivat davon, wie einem Hydrat, Hemihydrat oder Halbacetal, z.B. einem Halbacetal mit einem Niederalkanol, z.B. Methanol oder Aethanol, umsetzt und, wenn erwünscht, in einer erhältlichen Verbindung der Formel VI, worin $R_1$ durch Hydroxy substituiertes Niederalkyl ist, Hydroxy in geschütztes Hydroxy überführt.

Die Verbindung VI erhält man üblicherweise als Gemisch der beiden Isomeren (bezüglich der Gruppierung $>CH\sim OH$). Man kann aber auch die reinen Isomeren davon isolieren.

Die Anlagerung der Glyoxylsäureesterverbindung an das Stickstoffatom des Lactamrings findet bei Raumtemperatur oder, falls notwendig, unter Erwärmen, z.B. bis etwa 100°C, und zwar in Abwesenheit eines eigentlichen Kondensationsmittels und/oder ohne Bildung eines Salzes statt. Bei Verwendung des Hydrats der Glyoxylsäureverbindung wird Wasser gebildet, das, wenn notwendig, durch Destillation, z.B. azeotrop, oder durch Verwendung eines geeigneten Dehydrationsmittels, wie eines Molekularsiebs, entfernt wird. Vorzugsweise arbeitet man in Gegenwart eines geeigneten Lösungsmittels, wie z.B. Dioxan, Toluol oder Dimethylformamid, oder Lösungsmittelgemisches, wenn erwünscht oder notwendig, in der Atmosphäre eines Inertgases, wie Stickstoff.

Stufe 1.3: Verbindungen der Formel VII, worin $X_o$ für eine reaktionsfähige veresterte Hydroxygruppe, insbesondere für Halogen oder organisches Sulfonyloxy steht, werden hergestellt, indem man in einer Verbindung der Formel VI die sekundäre Hydroxygruppe in eine reaktionsfähige veresterte Hydroxygruppe, insbesondere in Halogen, z.B. Chlor oder Brom, oder in eine organische Sulfonyloxygruppe, wie Niederalkansulfonyloxy, z.B. Methansulfonyloxy, oder Arensulfonyloxy, z.B. Benzol- oder 4-Methylbenzolsulfonyloxy, umwandelt.

In den Ausgangsverbindungen der Formel VI steht $R_1$ vorzugsweise für durch eine geschützte Hydroxygruppe substituiertes Niederalkyl.

Die Verbindungen der Formel VII kann man in Form von Gemischen der Isomeren (bezüglich der Gruppierung $>CH{\sim}X_o$) oder in Form von reinen Isomeren erhalten.

Die obige Reaktion wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphoniumhalogenid, wie Triphenylphosphondibromid oder -dijodid, oder einem geeigneten organischen Sulfonsäurehalogenid, wie -chlorid, vorzugsweise in Gegenwart eines basischen, in erster Linie eines organischen basischen Mittels, wie eines aliphatischen tertiären Amins, z.B. Triäthylamin, Diisopropyläthylamin oder "Polystyrol-Hünigbase", oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, wenn notwendig, unter Kühlen und/oder in der Atmosphäre eines Inertgases, wie Stickstoff.

In einer so erhältlichen Verbindung der Formel VII kann eine reaktionsfähige veresterte Hydroxygruppe $X_o$ in an sich bekannter Weise in eine andere reaktionsfähige veresterte Hydroxygruppe umgewandelt werden. So kann man z.B. ein Chloratom durch Behandeln der entsprechenden Chlorverbindung mit einem geeigneten Bromid- oder Jodidsalz, wie Lithiumbromid oder -jodid, vorzugsweise in Gegenwart eines geeigneten Lösungsmittels, wie Aether, durch ein Brom- bzw. Jodatom austauschen.

Stufe 1.4: Das Ausgangsmaterial der Formel II' wird erhalten, indem man eine Verbindung der Formel VII, worin $X_o$ für eine reaktionsfähige veresterte Hydroxygruppe steht, mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt.

Die obige Reaktion wird vorzugsweise in Gegenwart eines geeigneten inerten Lösungsmittels, wie eines Kohlenwasserstoffs, z.B. Hexan, Cyclohexan, Benzol, Toluol oder Xylol, oder eines Aethers, z.B. Dioxan, Tetrahydrofuran oder Diäthylenglykol-dimethyläther, oder eines Lösungsmittelgemisches vorgenommen. Je nach Reaktionsfähigkeit arbeitet man unter Kühlen oder bei erhöhter Temperatur, etwa zwischen -10° und +100°, bevorzugt bei etwa 20° bis 80°, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man bei der Verwendung einer Phosphinverbindung üblicherweise in Gegenwart eines basischen Mittels, wie einer organischen Base, z.B. eines Amins, wie Triäthylamin, Diisopropyl-äthyl-amin oder "Polystyrol-Hünigbase", und gelangt so direkt zum Ylid-Ausgangsmaterial der Formel II (bzw. II'), das aus dem entsprechenden Phosphoniumsalz gebildet wird.

Eine Ausgangsverbindung der Formel II', worin $X^{\oplus}$ eine Phosphonogruppe zusammen mit einem Kation ist, wird vorzugsweise in situ hergestellt, indem man eine erhältliche Verbindung der Formel

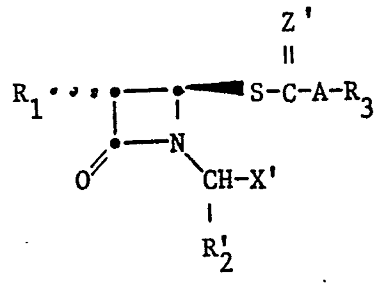

(IIa),

worin X' eine Phosphonogruppe bedeutet, mit einem geeigneten basischen Reagenz, wie einer anorganischen Base, z.B. einem Alkalimetallcarbonat, wie Natrium- oder Kaliumcarbonat, behandelt.

Stufe 1.4a

Eine Ausgangsverbindung der Formel II',worin Z' für Oxo steht, kann weiterhin erhalten werden, indem man ein Mercaptid der Formel VIII, worin M für ein Metallkation steht, mit einem den Rest $R_3$-A-C(=O)- einführenden Acylierungsmittel behandelt.

In dem Ausgangsmaterial der Formel VIII ist das Metallkation M beispielsweise ein Kation der Formel $M^+$ oder $M^{2+}/2$, wobei $M^+$ insbesondere für ein Silberkation und $M^{2+}$ z.B. für das zweiwertige Kation eines geeigneten Uebergangsmetalls, z.B. Kupfer, Blei oder Quecksilber, steht.

Ein den Rest $R_3$-A-C(=O)- einführendes Acylierungsmittel ist z.B. die Säure $R_3$-A-COOH oder ein reaktionsfähiges funktionelles Derivat davon, wie ein Säurehalogenid, z.B. Chlorid oder Bromid, Azid oder Anhydrid davon.

Die Acylierung erfolgt, wenn die freie Säure der Formel $R_3$-A-COOH eingesetzt wird, z.B. in Gegenwart eines geeigneten wasserentziehenden Mittels, wie eines Carbodiimids, z.B. N,N'-Dicyclohexylcarbodiimid, oder, wenn ein Säurederivat eingesetzt wird, in Gegenwart eines geeigneten säurebindenden Mittels, wie einer tertiären

aliphatischen oder aromatischen Base, z.B. Triäthylamin, Pyridin oder Chinolin, in einem inerten Lösungsmittel, wie einem chlorierten Kohlenwasserstoff, z.B. Methylenchlorid, oder einem Aether, z.B. Diäthyläther oder Dioxan, bei Raumtemperatur oder unter Erhitzen oder Kühlen, z.B. in einem Temperaturbereich von ca. -50° bis ca. +60°C, insbesondere bei ca. -30° bis ca. +20°C.

Die Ausgangsverbindungen der Formel VIII können beispielsweise hergestellt werden, indem man ein Azetidinon der Formel

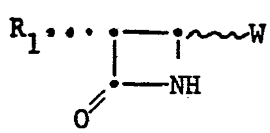

(V)

durch Umsetzen mit einem Alkalimetallsalz, z.B. dem Natriumsalz, einer Thioniederalkancarbonsäure, z.B. Thioessigsäure, oder des Triphenylmethylmercaptans in eine Verbindung der Formel

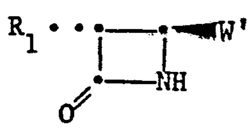

(V')

überführt, worin W' Triphenylmethylthio oder Niederalkanoylthio, z.B. Acetylthio, bedeutet, diese analog dem in den Reaktionsstufen 1.2, 1.3 und 1.4 beschriebenen Verfahren in eine Verbindung der Formel

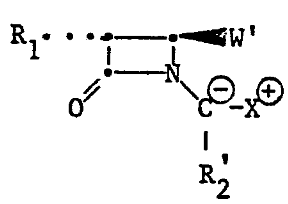

(X)

überführt und diese in Gegenwart einer Base, z.B. Pyridin oder Tri-n-butylamin, in einem geeigneten Lösungsmittel z.B. Diäthyläther oder Methanol, mit einem Salz der Formel MA, worin M die obige Bedeutung hat, insbesondere aber für ein Silber-Kation steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt.

Die Ylide der Formel II', worin Z' Sauerstoff oder Schwefel ist, können direkt in die Cyclisierungsreaktion zur Herstellung der Endprodukte der Formel I eingesetzt werden. Man kann aber auch in Verbindungen der Formel II', worin der Rest $R_1$ eine geschützte Hydroxygruppe, z.B. eine hydrolytisch leicht spaltbare geschützte Hydroxygruppe, wie trisubstituiertes Silyloxy, als Substituenten enthält, zuerst die Hydroxyschutzgruppe abspalten und dann die erhaltene Verbindung der Formel II', worin $R_1$ durch Hydroxy substituiertes Niederalkyl ist, in die Cyclisierungsreaktion einsetzen.

Stufe 1.5

Eine Verbindung der Formel (III) wird erhalten, in dem man ein Azetidinon der Formel (IV), worin Z' Schwefel bedeutet, mit einer Verbindung der Formel $R_2'$-COOH oder insbesondere einem reaktionsfähigen Derivat, wie einem Säurehalogenid, z.B. dem Säurechlorid, davon bei einer Temperatur von 20° bis 80°C, bevorzugt bei 40° bis 60°C, in einem inerten Lösungsmittel, wie einem der bei der Umsetzung von Verbindungen der Formel III zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, z.B. Triäthylamin, eines aromatischen Amins, z.B. Pyridin, oder insbesondere eines Alkalimetall- oder Erdalkalimetallcarbonats oder -hydrogencarbonats, z.B. Kaliumcarbonat oder Calciumcarbonat.

In den Verbindungen II', IV, VI und VII kann eine gegebenenfalls substituierte Methylidengruppe Z' durch Ozonisierung und anschliessender Reduktion des gebildeten Ozonids, gemäss dem nachfolgend in der Stufe 2.3 beschriebenen Verfahren, in die Oxogruppe Z überführt werden.

Ausgangsverbindungen der Formel V, worin W ein Sulfonylrest der Formel $R_o$-$SO_2$- ist, können auch nach dem folgenden Reaktionsschema II hergestellt werden.

Reaktionsschema II

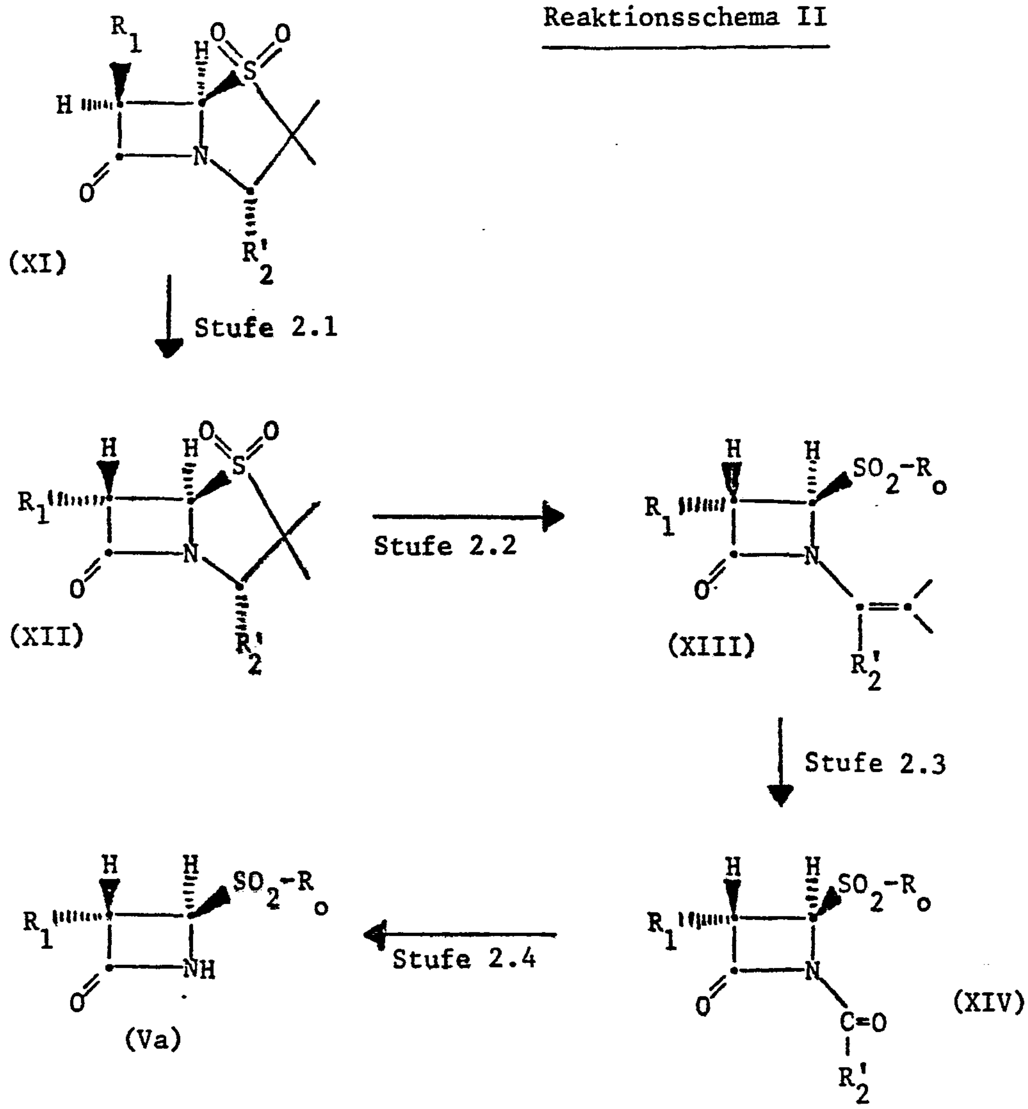


In den Verbindungen der Formel (XI) - (XIV) und (Va) steht $R_1$ insbesondere für durch eine geschützte Hydroxygruppe substituiertes Niederalkyl.

Stufe 2.1

Verbindungen der Formel (XII) können hergestellt werden, indem man eine Verbindung der Formel (XI) epimerisiert.

Die Epimerisierung erfolgt beispielsweise in Gegenwart eines basischen Mittels, wie eines Amins, z.B. eines Triniederalkylamins, z.B. Triäthylamin oder Aethyl-diisopropylamin, eines tertiären Amins, z.B. N,N-Dimethylanilin, eines aromatischen Amins, z.B. Pyridin, oder eines bicyclischen Amins, z.B. 1,5-Diazabicyclo[5,4,0]undec-5-en oder 1,5-Diazabicyclo[4,3,0]non-5-en, oder eines Alkalimetallniederalkanolats, z.B. Natriummethanolat, Natriumäthanolat oder Kalium-tert.-butanolat, in einem inerten Lösungsmittel, beispielsweise einem Aether, z.B. Diäthyläther, Dimethoxyäthan, Tetrahydrofuran oder Dioxan, Acetonitril oder Dimethylformamid, gegebenenfalls bei etwas erhöhter oder erniedrigter Temperatur, z.B. bei 0°C bis 50°C, vorzugsweise bei Raumtemperatur.

In den verfahrensgemäss erhältlichen Verbindungen der Formel (XII) kann eine im Rest $R_1$ enthaltende geschützte Hydroxygruppe durch eine andere geschützte Hydroxygruppe, beispielsweise eine hydrogenolytisch spaltbare geschützte Hydroxygruppe durch eine solvolytisch spaltbare geschützte Hydroxygruppe, ersetzt werden. Hydroxyschutzgruppen sind insbesondere die oben genannten, hydrogenolytisch abspaltbare Schutzgruppen beispielsweise wie angegeben substituiertes 1-Phenylniederalkyl oder Phenylniederalkoxycarbonyl, solvolytisch abspaltbare Schutzgruppen beispielsweise wie angegeben trisubstituiertes Silyl.

Die Umsetzung kann so durchgeführt werden, dass man zunächst die hydrogenolytisch abspaltbare Hydroxyschutzgruppe entfernt und in die entstandene Verbindung der Formel XII, worin $R_1$ durch Hydroxy substituiertes Niederalkyl ist, eine solvolytisch abspaltbare Hydroxyschutzgruppe einführt.

Die Abspaltung der hydrogenolytisch abspaltbaren Schutzgruppe erfolgt z.B. mit Wasserstoff oder einem Wasserstoffdonator, z.B. Cyclohexen oder Cyclohexadien, in Gegenwart eines

Hydrierungskatalysators, wie eines Palladiumkatalysators, z.B. Palladium auf Kohle, in einem inerten Lösungsmittel, wie einem halogenierten Kohlenwasserstoff, z.B. Methylenchlorid, einem Niederalkanol, z.B. Methanol oder Aethanol, einem Aether, z.B. Dioxan oder Tetrahydrofuran, oder auch in Wasser oder in Gemischen davon, bei einer Temperatur von etwa 0° bis etwa 80°C, vorzugsweise bei Raumtemperatur.
Die Abspaltung kann auch mit einem reduzierenden Metall, wie Zink, oder einer reduzierenden Metallegierung, z.B. Kupfer-Zink-Legierung, in Gegenwart eines Protonen-abgebenden Mittels, wie einer organischen Säure, z.B. Essigsäure, oder auch eines Niederalkanols, z.B. Aethanol, durchgeführt werden.

Die Einführung der solvolytisch abspaltbaren Hydroxyschutzgruppe erfolgt beispielsweise mit einer Verbindung der Formel $R'-X_3$, worin R' die Hydroxyschutzgruppe und $X_3$ z.B. eine reaktionsfähige veresterte Hydroxygruppe, beispielsweise Halogen, z.B. Chlor, Brom oder Jod, oder Sulfonyloxy, wie Methansulfonyloxy, Benzolsulfonyloxy oder 4-Toluolsulfonyloxy, bedeutet.

Ausgangsverbindungen der Formel (XI) sind beispielsweise aus der Deutschen Offenlegungsschrift 3 039 504 und aus der britischen Patentanmeldung 20 61 930 bekannt.

Stufe 2.2

Eine Verbindung der Formel (XIII) kann hergestellt werden, indem man eine Penam-Verbindung der Formel (XII) mit einem basischen Mittel und mit einem den Rest $R_o$ einführenden Veresterungsmittel behandelt.

Ein geeignetes basisches Mittel ist beispielsweise eines der unter Stufe 2.1 genannten basischen Mittel, insbesondere eines der genannten bicyclischen Amine, ferner auch ein Alkalimetallamid oder -hydrid, z.B. Natriumamid oder Natriumhydrid.

Ein Rest $R_o$ ist beispielsweise einer der unter Stufe 1.1 genannten organischen Reste, insbesondere gegebenenfalls substituiertes Niederalkyl, z.B. Methyl, Aethyl oder 2-Hydroxyäthyl, oder Benzyl.

Ein den Rest $R_o$ einführendes Veresterungsmittel ist z.B. eine Verbindung der Formel $R_o-X_4$, worin $X_4$ reaktionsfähiges verestertes Hydroxy, z.B. Halogen, wie Chlor, Brom oder Jod, oder Sulfonyloxy, wie Methansulfonyloxy, Benzolsulfonyloxy oder 4-Toluolsulfonyloxy, bedeutet. Zur Einführung eines 2-Hydroxyäthylrestes ist auch Aethylenoxid geeignet.

Die Umsetzung wird vorzugsweise zweistufig durchgeführt, wobei man in der ersten Stufe die Penam-Verbindung der Formel (XII) mit mindestens äquimolekularen Mengen des basischen Mittels behandelt und ein erhältliches Zwischenprodukt der Formel

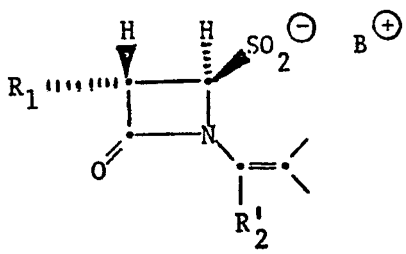

(XIIa)

worin $B^{\oplus}$ die protonierte Form (Kation) des basischen Mittels darstellt, vorzugsweise ohne Isolierung aus dem Reaktionsgemisch mit dem Veresterungsmittel umsetzt. Die Reaktion wird in einem inerten Lösungsmittel, beispielsweise einem Aether, z.B. Diäthyläther, Dimethoxyäthan, Tetrahydrofuran oder Dioxan, in Acetonitril, Dimethylformamid oder Hexamethylphosphorsäuretriamid, gegebenenfalls bei etwas erhöhter oder erniedrigter Temperatur, z.B. bei etwa 0°C bis 50°C, vorzugsweise bei Raumtemperatur, durchgeführt. In einer bevorzugten Ausführungsform des Verfahrens wird die Penam-Verbindung der Formel (XII) in situ hergestellt, indem man wie unter Stufe 2.1 beschrieben, eine

Verbindung der Formel (XI) zuerst mit katalytischen Mengen des basischen Mittels, z.B. 1,5-Diazabicyclo[5,4,0]undec-5-en, behandelt, und dann mit zumindest äquimolaren Mengen des gleichen basischen Mittels und des Veresterungsmittels weiter zu den Verbindungen der Formel (XIII) umsetzt.

Stufe 2.3

Ein Oxalyl-azetidinon der Formel (XIV) kann hergestellt werden, indem man eine Verbindung der Formel (XIII) ozonisiert und das gebildete Ozonid reduktiv zur Oxo-Verbindung spaltet.

Die Ozonisierung wird üblicherweise mit einem Ozon-Sauerstoff-Gemisch in einem inerten Lösungsmittel, wie einem Niederalkanol, z.B. Methanol oder Aethanol, einem Niederalkanon, z.B. Aceton, einem gegebenenfalls halogenierten Kohlenwasserstoff, z.B. einem Halogenniederalkan, wie Methylenchlorid oder Tetrachlorkohlenstoff, oder in einem Lösungsmittelgemisch, inkl. einem wässrigen Gemisch, vorzugsweise unter Kühlen, z.B. bei Temperaturen von etwa -80° bis etwa 0°C, durchgeführt.

Ein als Zwischenprodukt erhaltenes Ozonid wird, üblicherweise ohne isoliert zu werden, reduktiv zu einer Verbindung der Formel XIV gespalten, wobei man katalytisch aktivierten Wasserstoff, z.B. Wasserstoff in Gegenwart eines Schwermetallhydrierungskatalysators, wie eines Nickel-, ferner Palladiumkatalysators, vorzugsweise auf einem geeigneten Trägermaterial, wie Calciumcarbonat oder Kohle, oder chemische Reduktionsmittel, wie reduzierende Schwermetalle, inkl. Schwermetallegierungen oder -amalgame, z.B. Zink, in Gegenwart eines Wasserstoffdonators, wie einer Säure, z.B. Essigsäure, oder eines Alkohols, z.B. Niederalkanols, reduzierende anorganische Salze, wie Alkalimetalljodide, z.B. Natriumjodid, oder Alkalimetallhydrogensulfite, z.B. Natriumhydrogensulfit, in Gegenwart eines Wasserstoffdonators, wie einer Säure, z.B. Essigsäure, oder Wasser, oder reduzierende organische Verbindungen, wie Ameisensäure, verwendet. Als Reduktionsmittel können auch Verbindungen zum Einsatz kommen, die leicht in entsprechende Epoxidverbindungen oder Oxide umgewandelt werden können, wobei die Epoxidbildung aufgrund einer C,C-Doppelbindung und die Oxidbildung aufgrund eines vorhandenen Oxid-bildenden Hetero-, wie Schwefel-, Phosphor- oder Stickstoffatoms erfolgen kann. Solche Verbindungen sind z.B. geeignet substituierte Aethenverbindungen (die in der Reaktion in Aethylenoxidverbindungen umgewandelt werden), wie Tetracyanäthylen, oder insbesondere geeignete Sulfidverbindungen (die in der Reaktion in Sulfoxidverbindungen umgewandelt werden), wie Diniederalkylsulfide, in erster Linie Dimethylsulfid, geeignete organische Phosphorverbindungen, wie ein gegebenenfalls durch Phenyl und/oder Niederalkyl, z.B. Methyl, Aethyl, n-Propyl oder n-Butyl, substituiertes Phosphin (das in der Reaktion in ein Phosphinoxid umgewandelt wird), wie Triniederalkyl-phosphine, z.B. Tri-n-butylphosphin, oder Triphenylphosphin, ferner Triniederalkylphosphite (die in der Reaktion in Phosphorsäuretriniederalkylester übergeführt werden), üblicherweise in der Form von entsprechenden Alkoholadduktverbindungen, wie Trimethylphosphit, oder Phosphorigsäure-triamide, welche gegebenenfalls Niederalkyl als Substituenten enthalten, wie Hexaniederalkyl-phosphorigsäuretriamide, z.B. Hexamethylphosphorigsäuretriamid, letzteres vorzugsweise in der Form eines Methanoladdukts, ferner geeignete Stickstoffbasen (die in der Reaktion in die entsprechenden N-Oxide umgewandelt werden), wie heterocyclische Stickstoffbasen aromatischen Charakters, z.B. Basen vom Pyridintyp und insbesondere Pyridin selber. Die Spaltung des üblicherweise nicht isolierten Ozonids erfolgt normalerweise unter den Bedingungen, die man zu seiner Herstellung anwendet, d.h. in Gegenwart eines geeigneten Lösungsmittels oder Lösungsmittelgemisches,

sowie unter Kühlen oder leichtem Erwärmen, wobei man vorzugsweise bei Temperaturen von etwa -10°C bis etwa +25°C arbeitet und die Reaktion üblicherweise bei Raumtemperatur abschliesst.

Stufe 2.4

Ein Azetidinon der Formel (Va) kann hergestellt werden, indem man ein Oxalyl-azetidinon der Formel (XIV) solvolysiert.

Die Solvolyse kann als Hydrolyse, als Alkoholyse oder auch als Hydrazinolyse durchgeführt werden. Die Hydrolyse wird mit Wasser, gegebenenfalls in einem mit Wasser mischbaren Lösungsmittel, durchgeführt. Die Alkoholyse wird üblicherweise mit einem Niederalkanol, z.B. Methanol oder Aethanol, vorzugsweise in Gegenwart von Wasser und eines organischen Lösungsmittels, wie eines Niederalkancarbonsäure-niederalkylesters, z.B. Essigsäureäthylester, vorzugsweise bei Raumtemperatur, wenn notwendig unter Kühlen oder Erwärmen, z.B. bei einer Temperatur von etwa 0° bis etwa 80°C, durchgeführt. Die Hydrazinolyse wird auf konventionelle Weise mit einem substituierten Hydrazin, z.B. mit Phenyl- oder einem Nitrophenylhydrazin, wie 2-Nitrophenylhydrazin, 4-Nitrophenylhydrazin oder 2,4-Dinitrophenylhydrazin, das bevorzugt in etwa äquimolarer Menge eingesetzt wird, in einem organischen Lösungsmittel, wie einem Aether, z.B. Diäthyläther, einem aromatischen Kohlenwasserstoff, wie Benzol, einem halogenierten Kohlenwasserstoff, wie Methylenchlorid oder Chlorbenzol, einem Ester wie Aethylacetat, und dergleichen, bei Temperaturen zwischen etwa Raumtemperatur und etwa 65°C durchgeführt.

In einer bevorzugten Ausführungsform des Verfahrens geht man von einer Verbindung der Formel (XIII) aus, die wie angegeben ozonisiert und dann reduktiv zum Oxalyl-azetidinon der Formel (XIV) gespalten wird, welches ohne Isolierung aus dem Reaktionsgemisch weiter zum Azetidinon der Formel (Va) umgesetzt wird.

Bei der Ozonolyse entstehen gegebenenfalls geringe Mengen an Säure, welche die Abspaltung einer solvolytisch leicht abspaltbaren Hydroxyschutzgruppe im Rest $R_1$, z.B. eines trisubstituierten Silyl-Restes, bewirken können. Die dabei entstehende Verbindung der Formel

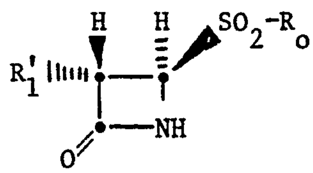

(Vb),

worin $R_1'$ durch Hydroxy substituiertes Niederalkyl bedeutet, kann, beispielsweise chromatographisch, vom geschützten Azetidinon (Va) abgetrennt und durch erneute Umsetzung mit dem die Hydroxyschutzgruppe R' einführenden Mittel der Formel $R'-X_3$ in das Azetidinon der Formel (Va) übergeführt werden.

In den Verbindungen der Formeln (II), (II'), (III), (VI), (VII) und (XII) bis (XIV) kann eine geschützte Carboxylgruppe $R_2'$ nach an sich bekannten Methoden in eine andere geschützte Carboxylgruppe $R_2'$ übergeführt werden, wobei unter Berücksichtigung der gebebenenfalls in diesen Verbindungen enthaltenen weiteren funktionellen Gruppen die gleichen Methoden zur Anwendung gelangen können, wie zur Umwandlung dieses Substituenten in den Verbindungen der Formel (I) angegeben ist.

Die Erfindung betrifft ebenfalls neue Ausgangsprodukte sowie verfahrensgemäss erhältliche neue Zwischenprodukte, wie diejenigen der Formel (II) bis (VIII), (incl. II', IIa, Va und Vb) sowie die angegebenen Verfahren zu ihrer Herstellung.

Vorzugsweise werden solche Ausgangsstoffe verwendet und die Reaktionsbedingungen so gewählt, dass man zu den vorstehend als besonders bevorzugt aufgeführten Verbindungen gelangt.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ Hydroxyniederalkyl bedeutet, $R_2$ Carboxyl oder eine unter physiologischen Bedingungen spaltbare veresterte Carboxylgruppe bedeutet und $R_3$ Amino, Niederalkylamino, Diniederalkylamino oder substituiertes Methylenamino ist, und pharmakologisch annehmbare Salze von solchen Verbindungen mit salzbildenden Gruppen haben antibakterielle Wirkungen. Beispielsweise sind sie in vitro gegen grampositive und gramnegative Kokken, z.B. Staphylococcus aureus, Streptococcus pyogenes, Streptococcus pneumoniae, Streptococcus faecalis, Neisseria meningitidis und Neisseria gonorrhoeae, gegen Enterobakterien, z.B. Escherichia coli, Proteus mirabilis und Klebsiella pneumoniae, gegen Haemophilus influenzae, Pseudomonas aeruginosa und Anaerobier, z.B. Bacteroides sp., und Clostridium sp. in minimalen Konzentrationen von ca. 0,02 bis ca. 64 µg/ml wirksam. In vivo, bei der systemischen Infektion der Maus, z.B. durch Staphylococcus aureus, Escherichia coli oder Streptococcus pyogenes, ergeben sich bei subkutaner oder oraler Applikation $ED_{50}$-Werte von ca. 0,3 bis ca. 100 mg/kg.

Die neuen Verbindungen können daher als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, wobei eine geschützte Carboxylgruppe von einer physiologisch spaltbaren veresterten Carboxylgruppe verschieden ist, können als Zwischenprodukte zur Herstellung der oben genannten pharmakologisch wirksamen Verbindungen der Formel I verwendet werden.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Kalziumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B Mannit, enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden

in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen von 125 mg bis etwa 5 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm,
IR: Infrarotspektrum,
UV: Ultraviolettspektrum,
NMR: Kernresonanzspektrum,
DBU: 1,5-Diazabicyclo[5.4.0]undec-5-en,
THF: Tetrahydrofuran,
DMF: Dimethylformamid.

Experimenteller Teil

Beispiel 1: (5R,6S)-2-[(2R,S)-2-(4-Nitrobenzyloxycarbonylamino)-prop-1-yl]-6-(tert.butyldimethylsilyloxymethyl)-2-penem-3-carbonsäure-p-nitrobenzylester

Eine Lösung von 6,27 g 2-[(3S,4R)-3-(tert.Butyldimethylsilyloxymethyl)-4-[(3R,S)-3-(4-nitrobenzyloxycarbonylamino)-butyroylthio]-2-oxo-azetidin-1-yl]-2-triphenylphosphoranylidenessigsäure-p-nitrobenzylester in 1 l Toluol wird unter Argonatmosphäre 12 h bei Rückflusstemperatur gerührt. Dann wird das Lösungsmittel abgedampft und das Rohprodukt durch Chromatographie an Silicagel gereinigt (Laufmittel Toluol/Aethylacetat 9:1). DC (Silicagel, Toluol/Aethylacetat 1:1): Rf = 0,56; IR ($CH_2Cl_2$): 3430; 1780; 1715; 1510; 1340; 1310 $cm^{-1}$.

Das Ausgangsmaterial kann wie folgt hergestellt werden:

a. (3R,S)-3-(4-Nitrobenzyloxycarbonylamino)-buttersäure

Zu einer Lösung von (3R,S)-3-Aminobuttersäure (20,6 g) in 41 ml Wasser und 90 ml 5 N NaOH-Lösung wird bei 0° fester Chlorameisensäure-4-nitrobenzylester (47,4 g) gegeben und die beige-farbene Suspension 16 Stunden bei Raumtemperatur nachgerührt. Nach dem Entfernen des Unlöslichen durch Filtration wird das Filtrat mit 100 ml Wasser verdünnt und zweimal mit $CH_2Cl_2$ gewaschen. Die wässrige Phase wird mit HCl 4 N auf pH 2 gestellt und zweimal mit $CH_2Cl_2$ extrahiert. Die vereinten organischen Extrakte werden einmal mit Sole gewaschen, über $MgSO_4$ getrocknet und zu den weissen Kristallen der Titelverbindung eingedampft. IR in $CH_2Cl_2$: 3425; 1710; 1500; 1335 $cm^{-1}$.

b. (3R,S)-3-(4-Nitrobenzyloxycarbonylamino)-thiobuttersäure

(3R,S)-3-(4-Nitrobenzyloxycarbonylamino)-buttersäure (14,1 g) wird in Methylenchlorid (250 ml) suspendiert und nach Abkühlen auf -10° durch Zugabe von 15,33 ml Triäthylamin gelöst. Bei gleicher Temperatur wird eine Lösung von Chlorameisensäureisobutylester (7,19 ml) in 50 ml Methylenchlorid zugegeben und bei -10° eine Stunde nachgerührt. Dann

wird während 2 Stunden $H_2S$ eingeleitet. Nach Entfernen des überschüssigen $H_2S$ mit Stickstoff wird das Reaktionsgemisch mit 2 N $H_2SO_4$ Lösung versetzt und gut geschüttelt. Die organische Phase wird abgetrennt und einmal mit 400 ml 1,5%iger $NaHCO_3$-Lösung extrahiert. Die wässrige Phase wird mit 2 N $H_2SO_4$ sauer gestellt, zweimal mit $CH_2Cl_2$ extrahiert und diese Extrakte anschliessend über $MgSO_4$ getrocknet. Nach Eindampfen der filtrierten organischen Lösung erhält man die Titelverbindung als gelbliches Oel. IR ($CH_2Cl_2$): 3430; 2580; 1715; 1500; 1345 $cm^{-1}$.

c. <u>(3S,4R)-3-(tert.-Butyl-dimethylsilyloxymethyl)-4-[(3R,S)-3-(4-nitrobenzyloxycarbonylamino)-butyroylthio]-azetidin-2-on</u>

8,22 g (3S,4R)-3-(tert.Butyldimethylsilyloxymethyl)-4-methylsulfonyl-azetidin-2-on und 11,7 g (3R,S)-3-(4-Nitrobenzyloxycarbonylamino)-thiobuttersäure werden in 180 ml $CH_2Cl_2$ gelöst. Die gelbliche Lösung wird erst mit 180 ml Wasser, dann mit 42 ml 1 N NaOH versetzt. Die kräftig gerührte Emulsion wird 1,5 h bei Raumtemperatur gerührt. Die organische Phase wird dann im Scheidetrichter abgetrennt und die wässrige noch zweimal mit $CH_2Cl_2$ extrahiert. Die vereinigten organischen Extrakte werden mit einer gesättigten wässrigen $NaHCO_3$-Lösung und dann mit Sole gewaschen, über $MgSO_4$ getrocknet und eingedampft. Das erhaltene Rohprodukt wird durch Chromatographie an Silicagel mit Toluol/Aethylacetat 9:1 gereinigt. DC (Silicagel, Aethylacetat): Rf = 0,65; IR ($CH_2Cl_2$): 3420; 1765; 1710; 1680; 1500; 1340 $cm^{-1}$.

Das Ausgangsmaterial (3S,4R)-3-(tert.-Butyldimethylsilyloxymethyl)-4-methylsulfonyl-azetidin-2-on kann wie folgt hergestellt werden:

ca. <u>(3S,5R,6R)-2,2-Dimethyl-6-(tert.-butyl-dimethylsilyloxymethyl)-penam-3-carbonsäuremethylester-1,1-dioxid</u>

Eine Lösung von 23,6 g (85 mMol) (3S,5R,6R)-2,2-Dimethyl-6-hydroxymethyl-penam-3-carbonsäuremethylester-1,1-dioxid in 50 ml Dimethylformamid wird mit 25,5 g (170 mMol) tert.-Butyl-dimethylchlorsilan und 11,5 g (170 mMol) Imidazol bei Raumtemperatur während 45 Minuten gerührt. Dann wird das Lösungsmittel am Hochvakuum abdestilliert und der Rückstand in Aethylacetat aufgenommen.Die Lösung wird mit 1N-Schwefelsäure und dann mit Wasser gewaschen und die wässerigen Lösungen zweimal mit Aethylacetat extrahiert. Die organische Phase wird mit Natriumsulfat getrocknet und am Rotationsverdampfer eingeengt. Das Produkt fällt als kristalline Masse an.

DC Silicagel, Toluol/Aethylacetat (4:1): Rf = 0,56

IR ($CH_2Cl_2$) 3,4; 5,57; 5,65 μm.

cb. 2-[(3S,4R)-3-(tert.-Butyl-dimethylsilyloxymethyl)-4-methylsulfonyl-2-oxoazetidin-1-yl]-3-methyl-2-butensäuremethylester

Eine Lösung von 202 g (0,51 Mol) (3R,5R,6R)-2,2-Dimethyl-6-(tert.-butyl-dimethylsilyloxymethyl)-penam-3-carbonsäuremethylester-1,1-dioxid in 800 ml Tetrahydrofuran werden mit 9 ml DBU versetzt und während 5 Minuten bei Raumtemperatur gerührt. Dann werden weitere 95 ml DBU zugegeben und 30 Minuten bei Raumtemperatur gerührt. Anschliessend fügte man unter Kühlung 42,3 ml (0,68 Mol) Methyljodid zu. Nach 3 Stunden Reaktionsdauer wird vom auskristallisierten DBU-Hydrojodid abfiltriert und das Filtrat eingeengt. Der Rückstand wird in Aethylacetat aufgenommen und die Lösung mit 1 N Schwefelsäure, Wasser und Natriumhydrogencarbonatlösung gewaschen. Die wässrigen Phasen werden zweimal mit Aethylacetat extrahiert. Die vereinigten organischen Phasen werden über Natriumsulfat getrocknet und die Lösung zu einem dicken Oel eingeengt.

DC [Silicagel, Toluol/Aethylacetat (4:1)]; Rf = 0,42

IR ($CH_2Cl_2$) 5,63; 5,81; 6,17 μm.

cc. (3S,4R)-3-Hydroxymethyl-4-methylsulfonyl-azetidin-2-on und (3S,4R)-3-(tert.-Butyl-dimethylsilyloxymethyl)-4-methylsulfonyl-azetidin-2-on

Eine Lösung von 25 g (61,7 mMol) 2-[(3S,4R)-3-(tert.-Butyl-dimethylsilyloxymethyl)-4-methylsulfonyl-2-oxo-azetidin-1-yl]-3-methyl-2-butensäuremethylester in 400 ml Methylenchlorid wird bei -10° mit einem Ozon/

Sauerstoffgemisch behandelt. Das Verschwinden des Ausgangsmaterials wird dünnschichtchromatographisch kontrolliert. Nach Beendigung der Reaktion werden 30 ml Dimethylsulfid zugegeben und für 3 Stunden bei Raumtemperatur weitergerührt. Die Lösung wird eingeengt und der Rückstand in einem Gemisch von 160 ml Methanol, 24 ml Aethylacetat und 3 ml Wasser aufgenommen und während 40 Minuten auf 70° erwärmt. Das Lösungsmittel wird anschliessend abgezogen und der Rückstand zweimal mit Toluol abgezogen. Das kristallisierende Oel wird in Methylenchlorid aufgenommen und die Kristalle, bestehend aus (3S,4R)-3-Hydroxymethyl-4-methylsulfonyl-azetidin-2-on, durch Filtration isoliert. Das Filtrat wird eingeengt und (3S,4R)-3-(tert.-Butyl-dimethylsilyloxymethyl)-4-methylsulfonyl-azetidin-2-on durch Chromatographie an Silicagel mit Toluol/Aethylacetat (3:1) in reiner Form erhalten:

<u>(3S,4R)-3-Hydroxymethyl-4-methylsulfonyl-azetidin-2-on:</u> DC, Silicagel, Toluol/Aethylacetat (1:1); Rf = 0,36, IR: ($CH_2Cl_2$) 2,96; 3,54, 5,65 μm.

<u>(3S,4R)-3-(tert.Butyl-dimethylsilyloxymethyl)-4-methylsulfonyl-azetidin-2-on</u>: DC; Silicagel, Toluol/Aethylacetat (1:1) Rf = 0,06.

Eine Lösung von 14,6 g (81,5 mMol) (3S,4R)-3-Hydroxymethyl-4-methylsulfonyl-azetidin-2-on in 40 ml Dimethylformamid wird mit 24 g (183 mMol) tert.-Butyldimethylchlorsilan und 11 g (163 mMol) Imidazol während 45 Minuten bei Raumtemperatur versetzt. Dann wird das Lösungsmittel am Hochvakuum abgezogen und der Rückstand in Aethylacetat aufgenommen. Die organische Phase wird nacheinander mit 1N-Schwefelsäure, Wasser und Natriumbicarbonatlösung gewaschen. Die wässerigen Phasen werden zweimal mit Aethylacetat extrahiert. Die vereinigten organischen Phasen werden über Natriumsulfat getrocknet und am Rotationsverdampfer eingeengt. Der kristalline Rückstand ist reines (3S,4R)-3-(tert.-Butyl-dimethylsilyloxymethyl)-4-methylsulfonyl-azetidin-2-on.

d. 2-[(3S,4R)-3-(tert.-Butyl-dimethylsilyloxymethyl)-4-[(3R,S)-3-(4-nitrobenzyloxycarbonylamino)-butyroylthio]-2-oxo-azetidin-1-yl]-2-hydroxyessigsäure-p-nitrobenzylester

Ein Gemisch von 10,23 g (3S,4R)-3-(tert.Butyldimethylsilyloxymethyl)-4-[(3R,S)-3-(4-nitrobenzyloxycarbonylamino)-butyroylthio]-azetidin-2-on und 10,21 g Glyoxylsäure-p-nitrobenzylester-äthylhemiacetal in 170 ml Toluol und 10 ml abs. DMF wird mit 80 g Molekularsieb (4 Å) versetzt und während 16 h bei Raumtemperatur und anschliessend 2 h bei 50° gerührt. Das Gemisch wird filtriert und der Filterrückstand mit Toluol nachgewaschen. Eindampfen des Filtrats und Trocknung im Hochvakuum bei 50° ergibt das Produkt als gelbes Oel. DC (Silicagel, Aethylacetat): Rf = 0,67; IR ($CH_2Cl_2$): 3430; 1765; 1730; 1690; 1505; 1340 $cm^{-1}$.

e. 2-[(3S,4R)-3-(tert.-Butyl-dimethylsilyloxymethyl)-4-[(3R,S)-3-(4-nitrobenzyloxycarbonylamino)-butyroylthio]-2-oxo-azetidin-1-yl]-2-triphenylphosphoranylidenessigsäure-p-nitrobenzylester

Zu einer Lösung von 13,7 g 2-[(3S,4R)-3-(tert.Butyldimethylsilyloxymethyl)-4-[(3R,S)-3-(4-nitrobenzyloxycarbonylamino)-butyroylthio]-2-oxo-azetidin-1-yl]-2-hydroxyessigsäure-p-nitrobenzylester in 250 ml abs. Tetrahydrofuran werden unter Rühren bei 0° nacheinander 2,03 ml Thionylchlorid und 2,3 ml Pyridin zugegeben. Die weisse Suspension wird noch bei 0° während 30 Minuten gerührt und filtriert. Nach Eindampfen wird der erhaltene gelbe Schaum in 100 ml Dioxan gelöst und mit [illegible],1 ml 2,6-Dimethylpyridin und 6,98 g Triphenylphosphin versetzt. Nach 17stündigem Rühren bei 40° werden die Feststoffe abgetrennt und das Filtrat wird eingedampft. Das Rohprodukt wird durch Chromatographie an Silicagel (Laufmittel Toluol-Aethylacetat 9:1) gereinigt. DC (Silicagel, Toluol/Aethylacetat 1:1): Rf = 0,36; IR ($CH_2Cl_2$): 3430; 1745; 17[illegible]; 1615; 1510; 1430 $cm^{-1}$.

Beispiel 2: (5R,6S)-2-[(2R,S)-2-(4-Nitrobenzyloxycarbonylamino)-prop-1-yl]-6-hydroxymethyl-2-penem-3-carbonsäure-p-nitrobenzylester

Eine Lösung von 3,14 g (5R,6S)-2-[(2R,S)-2-(4-Nitrobenzyloxycarbonylamino)-prop-1-yl]-6-(tert.butyldimethylsilyloxymethyl)-2-penem-3-carbonsäure-p-nitrobenzylester in 50 ml abs. THF wird nacheinander mit 1,83 ml Essigsäure und 80 ml einer 0,1 N Tetrabutylammoniumfluorid-Lösung in THF versetzt, Nach 4,5 h Rühren wird mit 1,4 l $CH_2Cl_2$ verdünnt und mit 200 ml einer gesättigten $NaHCO_3$ in $H_2O$ Lösung gewaschen. Die organische Phase wird dann mit Sole gewaschen, über $MgSO_4$ getrocknet und nach Abfiltrieren eingedampft. Das Rohprodukt wird durch Chromatographie auf Silicagel gereinigt (Laufmittel: ab Toluol/Aethylacetat 1:1 bis Aethylacetat absolut). DC (Silicagel, Aethylacetat): Rf = 0,46; IR ($CH_2Cl_2$): 3600; 3430, 1780; 1715; 1515; 1340 $cm^{-1}$.

Beispiel 3: (5R,6S)-2-[(2R,S)-2-Aminoprop-1-yl]-6-hydroxymethyl-2-penem-3-carbonsäure

229 mg (5R,6S)-2-[(2R,S)-2-(4-Nitrobenzyloxycarbonylamino)-prop-1-yl]-6-hydroxymethyl-2-penem-3-carbonsäure-p-nitrobenzylester werden in 30 ml Aethylacetat, 12 ml THF und 24 ml Wasser mit 0,15 g Pd/C (10%) versetzt und bei Raumtemperatur und Normaldruck während 2 h hydriert. Der Katalysator wird abfiltriert und das Reaktionsgemisch wird nochmals mit 0,1 g Pd/C (10%) und dann 4 ml 0,1 N HCl versetzt und 1 h weiter hydriert. Der Katalysator wird abfiltriert, die wässrige Phase abgetrennt und die organische Phase nochmals mit Wasser extrahiert. Die vereinigten wässrigen Extrakte werden mit 1 Aequivalent $NaHCO_3$ versetzt und mit Aethylacetat gewaschen. Anschliessend wird die wässrige Phase lyophilisiert. DC (Reversed Phase Opti-$UPC_{12}$) in Wasser: Rf = 0,13; UV (Phosphatpuffer pH 7,4): $\lambda_{max}$ = 304 nm.

Beispiel 4: (5R,6S)-2-[2-Methyl-2-(4-nitrobenzyloxycarbonylamino)-prop-1-yl]-6-(tert.-butyldimethylsilyloxymethyl)-2-penem-3-carbonsäure-p-nitrobenzylester

Analog Beispiel 1 werden 1,51 g 2-[(3S,4R)-3-(tert.-Butyldimethylsilyloxymethyl)-4-[3-methyl-3-(4-nitrobenzyloxycarbonylamino)-butyroylthio]-2-oxo-azetidin-1-yl]-2-triphenylphosphoranylidenessigsäure-p-nitrobenzylester in 250 ml Toluol nach 46 h Rühren bei Rückflusstemperatur in die Titelverbindung überführt. DC (Silicagel, Toluol/Aethylacetat 1:1): Rf = 0,6; IR ($CH_2Cl_2$): 3430; 1775; 1710; 1510; 1340; 1300 $cm^{-1}$.

Das Ausgangsmaterial kann wie folgt hergestellt werden:

a. 3-Methyl-3-(4-nitrobenzyloxycarbonylamino)-buttersäure

Analog Beispiel 1a werden 7,79 g 3-Methyl-3-aminobuttersäure zur Titelverbindung umgesetzt. IR in $CH_2Cl_2$: 3430, 1710, 1500, 1340 $cm^{-1}$.

b. 3-Methyl-3-(4-nitrobenzyloxycarbonylamino)-thiobuttersäure

Analog Beispiel 1b werden 9,5 g 3-Methyl-3-(4-nitrobenzyloxycarbonylamino)-buttersäure in die Titelverbindung überführt. IR ($CH_2Cl_2$): 3420; 2570; 1710; 1490; 1340 $cm^{-1}$.

c. (3S,4R)-3-(tert.-Butyldimethylsilyloxymethyl)-4-[3-methyl-3-(4-nitrobenzyloxycarbonylamino)-butyroylthio]-azetidin-2-on

Analog Beispiel 1c werden 5,98 g 3-Methyl-3-(4-nitrobenzyloxycarbonylamino)-thiobuttersäure und 3,75 g (3S,4R)-3-(tert.Butyldimethylsilyloxymethyl)-4-methylsulfonyl-azetidin-2-on in die Titelverbindung überführt. DC (Silicagel, Toluol-Aethylacetat 1:1): Rf = 0,44; IR ($CH_2Cl_2$): 3400; 1765; 1715; 1675; 1490; 1335 $cm^{-1}$.

d. 2-[(3S,4R)-3-(tert.-Butyldimethylsilyloxymethyl)-4-[3-methyl-3-(4-nitrobenzyloxycarbonylamino)-butyroylthio]-2-oxo-azetidin-1-yl]-2-hydroxyessigsäure-p-nitrobenzylester

Analog Beispiel 1d werden 3,75 g (3S,4R)-3-(tert.-Butyldimethylsilyloxy-

methyl)-2-[3-methyl-3-(4-nitrobenzyloxycarbonylamino)-butyroylthio]-azetidin-2-on und 3,57 g Glyoxylsäure-p-nitrobenzylester-äthylhemiacetal in die Titelverbindung überführt. DC (Silicagel, Toluol-Aethylacetat): Rf = 0,43 und 0,36; IR: ($CH_2Cl_2$): 3420; 1760; 1735; 1710; 1680; 1505; 1335 $cm^{-1}$.

e. 2-[(3S,4R)-3-(tert.-Butyldimethylsilyloxymethyl)-4-[3-methyl-3-(4-nitrobenzyloxycarbonylamino)-butyroylthio]-2-oxo-azetidin-1-yl]-2-triphenylphosphoranylidenessigsäure-p-nitrobenzylester

Analog Beispiel 1e werden 2,32 g 2-[(3S,4R)-3-(tert.-Butyldimethylsilyloxymethyl)-4-[3-methyl-3-(4-nitrobenzyloxycarbonylamino)-butyroylthio]-2-oxo-azetidin-1-yl]-2-hydroxyessigsäure-p-nitrobenzylester zur Titelverbindung umgesetzt. DC (Silicagel, Toluol/Aethylacetat 1:1) Rf = 0,47; IR ($CH_2Cl_2$): 3420; 1740; 1730; 1675; 1610; 1600; 1500; 1335 $cm^{-1}$.

Beispiel 5: (5R,6S)-2-[2-Methyl-2-(4-nitrobenzyloxycarbonylamino)-prop-1-yl]-6-hydroxymethyl-2-penem-3-carbonsäure-p-nitrobenzylester

Analog Beispiel 2 werden 2,29 g (5R,6S)-2-[2-Methyl-2-(4-nitrobenzyloxycarbonylamino)-prop-1-yl]-6-(tert.butyldimethylsilyloxymethyl)-2-penem-3-carbonsäure-p-nitrobenzylester zur Titelverbindung umgesetzt. DC (Silicagel, Toluol/Aethylacetat): Rf = 0,23. IR ($CH_2Cl_2$): 3600; 3430; 1780; 1710; 1510; 1345 $cm^{-1}$.

Beispiel 6: (5R,6S)-2-(2-Amino-2-methyl-prop-1-yl)-6-hydroxymethyl-2-penem-3-carbonsäure

Analog Beispiel 3 werden 0,7 g (5R,6S)-2-[2-Methyl-2-(4-nitrobenzyloxycarbonylamino)-prop-1-yl]-6-hydroxymethyl-2-penem-3-carbonsäure-p-nitrobenzylester zur Titelverbindung umgesetzt. DC (Reversed Phase Opti $UPC_{12}$) in Wasser: Rf = 0,15. UV (Phosphatpuffer pH 7,4): $\lambda_{max}$ = 307 nm.

Beispiel 7: (5R,6S)-2-[(3R,S)-3-(4-Nitrobenzyloxycarbonylamino)-but-1-yl]-6-(tert.-butyldimethylsilyloxymethyl)-2-penem-3-carbonsäure-p-nitrobenzylester

Analog Beispiel 1 werden 2,3 g 2-[(3S,4R)-3-(tert.-Butyldimethylsilyloxymethyl)-4-[(4R,S)-4-(4-nitrobenzyloxycarbonylamino)-valeroylthio]-2-oxo-azetidin-1-yl]-2-triphenylphosphoranylidenessigsäure-p-nitrobenzylester in die Titelverbindung überführt. DC (Silicagel, Toluol/Aethylacetat 1:1): Rf = 0,49.

Das Ausgangsmaterial kann wie folgt hergestellt werden:

a. (4R,S)-4-(4-Nitrobenzyloxycarbonylamino)-valeriansäure

Analog Beispiel 1a werden 9,5 g(4R,S)-4-Aminovaleriansäure zur Titelverbindung umgesetzt. NMR ($DMSO-d_6$):$\delta$= 12,0 (br; 1H), 8,3 (m; 2H), 7,7 (m; 2H), 7,25 (br; 1H), 5,2 (s; 2H), 3,6 (m; 1H), 2,25 (t; 2H), 1,7 (m; 2H), 1,1 ppm (d; 3H).

b. (4R,S)-4-(4-Nitrobenzyloxycarbonylamino)-thiovaleriansäure

Analog Beispiel 1b werden 15 g (4R,S)-4-(4-Nitrobenzyloxycarbonylamino)-valeriansäure in die Titelverbindung überführt. IR ($CH_2Cl_2$): 3430; 2570; 1715; 1505; 1345 $cm^{-1}$.

c. (3S,4R)-3-(tert.-Butyldimethylsilyloxymethyl)-4-[(4R,S)-4-(4-nitrobenzyloxycarbonylamino)-valeroylthio]-azetidin-2-on

Analog Beispiel 1c werden 11,4 g (4R,S)-4-(4-Nitrobenzyloxycarbonylamino)-thiovaleriansäure und 8,31 g (3S,4R)-3-(tert.-Butyldimethylsilyloxymethyl)-4-methylsulfonyl-azetidin-2-on in die Titelverbindung überführt. DC (Silicagel, Toluol/Aethylacetat 1:1); Rf = 0,14.

d. 2-[(3S,4R)-3-(tert.-Butyldimethylsilyloxymethyl)-4-[(4R,S)-4-(4-nitrobenzyloxycarbonylamino)-valeroylthio]-2-oxo-azetidin-1-yl]-2-hydroxyessigsäure-p-nitrobenzylester

Analog Beispiel 1d werden 11,18 g (3S,4R)-3-tert.-Butyldimethylsilyloxymethyl)-4-[(4R,S)-4-(4-nitrobenzyloxycarbonylamino)-valeroylthio]-azetidin-2-on und 8,14 g Glyoxylsäure-p-nitrobenzylester-äthylhemiacetal in die Titelverbindung überführt. DC (Silicagel, Toluol/Aethylacetat 1:1); Rf = 0,25 und 0,28.

e. 2-[(3S,4R)-3-(tert.-Butyldimethylsilyloxymethyl)-4-[(4R,S)-4-(4-nitrobenzyloxycarbonylamino)-valeroylthio]-2-oxo-azetidin-1-yl]-2-triphenylphosphoranylidenessigsäure-p-nitrobenzylester

Analog Beispiel 1e werden 5,2 g 2-[(3S,4R)-3-(tert.-Butyldimethylsilyloxymethyl)-4-[(4R,S)-4-(4-nitrobenzyloxycarbonylamino)-valeroylthio]-2-oxo-azetidin-1-yl]-2-hydroxyessigsäure-p-nitrobenzylester zur Titelverbindung umgesetzt. DC (Silicagel, Toluol/Aethylacetat 1:1): Rf = 0,32.

Beispiel 8: (5R,6S)-2-[(3R,S)-3-(4-Nitrobenzyloxycarbonylamino)-but-1-yl]-6-hydroxymethyl-2-penem-3-carbonsäure-p-nitrobenzylester

Analog Beispiel 2 werden 1,34 g (5R,6S)-2-[(3R,S)-3-(4-Nitrobenzyloxycarbonylamino)-but-1-yl]-6-(tert.-butyldimethylsilyloxymethyl)-2-penem-3-carbonsäure-p-nitrobenzylester in die Titelverbindung umgesetzt. IR ($CH_2Cl_2$): 3600; 3420; 1780; 1715; 1510; 1340 $cm^{-1}$.

Beispiel 9: (5R,6S)-2-[(3R,S)-3-Amino-but-1-yl]-6-hydroxymethyl-2-penem-3-carbonsäure

Analog Beispiel 3 werden 0,82 g (5R,6S)-2-[(3R,S)-3-(4-Nitrobenzyloxycarbonylamino)-but-1-yl]-6-hydroxymethyl-2-penem-3-carbonsäure-p-nitrobenzylester zur Titelverbindung umgesetzt. UV (Phosphatpuffer pH 7,4): $\lambda_{max}$ = 305 nm.

Beispiel 10: (5R,6S)-2-[(2R,S)-1-(4-Nitrobenzyloxycarbonylamino)-prop-2-yl]-6-(tert.-butyldimethylsilyloxymethyl)-2-penem-3-carbonsäure-p-nitrobenzylester

Analog Beispiel 1 werden 1,13 g 2-[(3S,4R)-3-(tert.-Butyl-dimethylsilyloxymethyl)-4-[(2R,S)-2-methyl-3-(4-nitrobenzyloxycarbonylamino)-propionylthio]-2-oxo-azetidin-1-yl]-2-phosphoranylidenessigsäure-p-nitrobenzylester in die Titelverbindung überführt. DC (Silicagel, Toluol/Aethylacetat 1:1): Rf = 0,4.

Das Ausgangsmaterial kann wie folgt hergestellt werden.

a. (2R,S)-2-Methyl-3-(4-nitrobenzyloxycarbonylamino)-propionsäure

Analog Beispiel 1a werden 5,69 g (2R,S)-3-Amino-2-methyl-propionsäure zur Titelverbindung umgesetzt. IR ($CH_2Cl_2$): 3450; 1720; 1510; 1345 $cm^{-1}$.

b. (2R,S)-2-Methyl-3-(4-nitrobenzyloxycarbonylamino)-thiopropionsäure

Analog Beispiel 1b werden 10,4 g (2R,S)-2-Methyl-3-(4-nitrobenzyloxycarbonylamino)-propionsäure zur Titelverbindung umgesetzt. IR ($CH_2Cl_2$): 4440; 2570; 1715; 1690; 1510; 1345 $cm^{-1}$.

c. (3S,4R)-3-(tert.-Butyl-dimethylsilyloxymethyl)-4-[(2R,S)-2-methyl-3-(4-nitrobenzyloxycarbonylamino)-propionylthio]-azetidin-2-on

Analog Beispiel 1c werden 9,27 g (2R,S)-2-Methyl-3-(4-nitrobenzyloxycarbonylamino)-thiopropionsäure und 6,07 g (3S,4R)-3-(tert.Butyldimethylsilyloxymethyl)-4-methylsulfonyl-azetidin-2-on in die Titelverbindung überführt. DC (Silicagel, Toluol/Aethylacetat 1:1): Rf = 0,34; IR ($CH_2Cl_2$): 3440; 3400; 1765; 1710; 1675; 1500; 1335 $cm^{-1}$.

d. 2-[(3S,4R)-3-(tert.-Butyl-dimethylsilyloxymethyl)-4-[(2R,S)-2-methyl-3-(4-nitrobenzyloxycarbonylamino)-propionylthio]-2-oxo-azetidin-1-yl]-2-hydroxyessigsäure-p-nitrobenzylester

Analog Beispiel 1d werden 0,98 g (3S,4R)-3-(tert.-Butyldimethylsilyloxymethyl)-4-[(2R,S)-2-methyl-3-(4-nitrobenzyloxycarbonylamino)-propionylthio]-azetidin-2-on in die Titelverbindung überführt. DC (Silicagel, Toluol/Aethylacetat 1:1): Rf = 0,5; IR ($CH_2Cl_2$): 3510; 3420; 1765; 1740; 1715; 1690 $cm^{-1}$.

e. 2-[(3S,4R)-3-(tert.-Butyl-dimethylsilyloxymethyl)-4-[(2R,S)-2-methyl-3-(4-nitrobenzyloxycarbonylamino)-propionylthio]-2-oxo-azetidin-1-yl]-2-phosphoranyliden-essigsäure-p-nitrobenzylester

Analog Beispiel 1e werden 2,6 g 2-[(3S,4R)-3-(tert.-Butyldimethylsilyloxymethyl)-4-[(2R,S)-2-methyl-3-(4-nitrobenzyloxycarbonylamino)-propionylthio]-2-oxo-azetidin-1-yl]-2-hydroxyessigsäure-p-nitrobenzylester in die Titelverbindung überführt. DC (Silicagel, Toluol/Aethylacetat 1:1): Rf = 0,36; IR ($CH_2Cl_2$): 3440, 1745; 1715; 1675; 1615; 1600 $cm^{-1}$.

Beispiel 11: (5R,6S)-2-[(2R,S)-1-(4-Nitrobenzyloxycarbonylamino)-prop-2-yl]-6-hydroxymethyl-2-penem-3-carbonsäure-p-nitrobenzylester

Analog Beispiel 2. werden 0,28 g (5R,6S)-2-[(2R,S)-1-(4-Nitrobenzyloxycarbonylamino)-prop-2-yl]-6-(tert.butyldimethylsilyloxymethyl)-2-penem-3-carbonsäure-p-nitrobenzylester in die Titelverbindung überführt. DC (Silicagel, Toluol/Aethylacetat 1:1):Rf = 0,17; IR ($CH_2Cl_2$): 3440; 1775; 1715; 1510; 1340 $cm^{-1}$.

Beispiel 12: (5R,6S)-2-[(2R,S)-1-Aminoprop-2-yl]-6-hydroxymethyl-2-penem-3-carbonsäure

Analog Beispiel 3 werden 750 mg (5R,6S)-2-[(2R,S)-1-(4-Nitrobenzyloxycarbonylamino)-prop-2-yl]-6-hydroxymethyl-2-penem-3-carbonsäure-p-nitrobenzylester in die Titelverbindung überführt. UV (Phosphatpuffer pH 7,4): $\lambda_{max}$ = 301 nm.

Beispiel 13: Natriumsalz der (5R,6S)-2-[(2R,S)-2-(1-Aethoxycarbonylprop-1-en-2-ylamino)-prop-1-yl]-6-hydroxymethyl-2-penem-3-carbonsäure

0,139 ml (1,1 mMol) Acetessigsäureäthylester werden zu einer Suspension von 0,257 mg (1 mMol) (5R,6S)-2-[(2R,S)-2-Aminoprop-1-yl]-6-hydroxymethyl-2-penem-3-carbonsäure und 0,5 ml 2N-Natronlauge in 3 ml Isopropanol gegeben und 3 Stunden bei Raumtemperatur gerührt. Durch Zugabe von Diäthyläther wird das Produkt ausgefällt. IR-Spektrum (Nujol): Absorptionsbanden bei 3330; 1791; 1739 $cm^{-1}$.

Beispiel 14: (5R,6S)-2-[(2R,S)-1-Allyloxycarbonylamino-prop-2-yl]-6-(tert.butyldimethylsilyloxymethyl)-2-penem-3-carbonsäure-allylester.

Eine Lösung von 0,9 g 2-[(3S,4R)-3-(tert.Butyldimethylsilyloxymethyl)-4-[(2R,S)-2-methyl-3-allyloxycarbonylamino-propionylthio]-2-oxo-azetidin-1-yl]-2-triphenylphosphoranylidenessigsäure-allylester in 150 ml abs. Toluol wird unter Argonatmosphäre 24 Stunden bei Rückfluss-Temperatur gerührt. Dann wird das Lösungsmittel eingedampft und das Rohprodukt durch Chromatographie an Silicagel gereinigt. (Laufmittel Toluol-Aethylacetat 9:1). IR ($CH_2Cl_2$): 3440, 1780, 1710 $cm^{-1}$.

Das Ausgangsmaterial kann wie folgt hergestellt werden:

a. (3S,4R)-3-(tert.-Butyl-dimethylsilyloxymethyl)-4-triphenylmethylthio-azetidin-2-on

12,5 g Triphenylmethylmercaptan werden in 70 ml Methanol bei 0° suspendiert, und über 10 Minuten portionsweise mit insgesamt 2,2 g einer 50 %igen Natriumhydrid-Suspension in Oel versetzt. Anschliessend wird eine Emulsion von 11,1 g (3S,4R)-3-(tert.-Butyldimethylsilyloxymethyl)-4-methylsulfonyl-azetidin-2-on in 70 ml Aceton und 70 ml Wasser über 30 Minuten zugetropft. Nach 30 Minuten Rühren bei 0° und 1 Stunde bei Raumtemperatur wird das Reaktionsgemisch am Rotationsverdampfer eingeengt, mit Methylenchlorid versetzt, und die wässrige Phase wird abgetrennt. Die organische Lösung wird mit Sole gewaschen und über Natriumsulfat getrocknet. Nach Einengen wird die rohe Titelverbindung durch Chromathographie auf Silicagel (Laufmittel Toluol/Essigester 19:1) gereinigt. DC (Toluol-Essigester 19:1): $R_f$ = 0,64; IR (Methylenchlorid): 3390, 1760, 1117, 835cm$^{-1}$.

b. 2-[(3S,4R)-3-(tert.-Butyl-dimethylsilyloxymethyl)-4-triphenylmethylthio-2-oxo-azetidin-1-yl]-2-hydroxyessigsäure-allylester

8,4 g (3S,4R)-3-(tert.Butyldimethylsilyloxymethyl)-4-triphenylmethylthio-azetidin-2-on und 8,23 g (Glyoxylsäureallylester-ethylhemiacetal in 170 ml abs. Toluol werden mit 27 g Molekularsieb (4 Å) versetzt und während 10 Stunden bei 55° gerührt. Nach Abfiltrieren und Einengen am Rotationsverdampfer unter vermindertem Druck wird das Rohprodukt durch Chromatographie an Silicagel gereinigt. (Laufmittel Toluol/Aethylacetat 95:5). DC (Silicagel, Toluol/Aethylacetat 10:1): $R_f$ = 0,37 und 0,27; IR ($CH_2CH_2$): 3520, 1760, 1745 cm$^{-1}$.

c. 2-[(3S,4R)-3-(tert.-Butyl-dimethylsilyloxymethyl)-4-triphenylmethylthio-2-oxo-azetidin-1-yl]-2-triphenylphosphoranyliden-essigsäure-allylester

Zu einer Lösung von 604 mg 2-[(3S,4R)-3-(tert.-Butyldimethylsilyloxymethyl)-4-triphenylmethylthio-2-oxo-azetidin-1-yl]-2-hydroxyessigsäure-allylester in 5 ml Tetrahydrofuran werden unter Rühren bei -15° nacheinander 80 µl Thionylchlorid und 88 µl Pyridin innert 5 Minuten zugegeben. Die weisse Suspension wird 1 Stunde bei -10° nachgerührt und über Hyflo filtriert. Nach Waschen des Rückstands mit Toluol wird am Rotationsverdampfer eingeengt. Der Rückstand wird in 3 ml Dioxan gelöst, mit 293 mg Triphenylphosphin und 0,13 ml 2,6-Lutidin versetzt und während 2 Std. bei 115° Badtemperatur gerührt. Das Gemisch wird über Hyflo filtriert und der Rückstand mit Toluol nachgewaschen. Die vereinten Filtrate werden eingedampft. Die Chromatographie des Rückstandes an Silicagel ergibt das reine Produkt (Laufmittel Toluol/Aethylacetat 95:5). DC (Silicagel, Toluol/Aethylacetat 1:1): $R_f$ = 0,18; IR ($CH_2Cl_2$): 1745, 1605 $cm^{-1}$.

d. <u>Silbersalz des 2-[(3S,4R)-3-(tert.-Butyldimethylsilyloxymethyl)-4-mercapto-2-oxo-azetidin-1-yl]-2-triphenylphosphoranyliden-essigsäure-allylesters</u>

7,5 g 2-[(3S,4R)-3-(tert.Butyl-dimethylsilyloxymethyl)-4-triphenylmethylthio-2-oxo-azetidin-1-yl]-2-triphenylphosphoranylidenessigsäure-allylester werden in 87 ml Aether vorgelegt und bei Raumtemperatur mit 70 ml einer 0,5 M wässrigen Silbernitrat-Lösung versetzt. Danach gibt man tropfenweise ein Gemisch aus 3,6 ml Tributylamin, 0,18 ml Trifluoressigsäure und 25 ml Aether dazu und rührt das Reaktionsgemisch während 20 Minuten nach. Dann wird der Feststoff abgenutscht und mit Aether, Wasser und nochmals Aether gewaschen. Der Feststoff wird schliesslich zur Reinigung nochmals in 40 ml Aether und 40 ml Wasser aufgeschlemmt, abgenutscht und getrocknet. IR ($CH_2Cl_2$): 1760, 1620 $cm^{-1}$.

e. <u>2-[(3S,4R)-3-(tert.-Butyl-dimethylsilyloxymethyl)-4-[(2R,S)-2-methyl-3-allyloxycarbonylamino-propionylthio]-2-oxo-azetidin-1-1-yl]-2-triphenylphosphoranylidenessigsäureallylester.</u>

5 g Silbersalz des 2-[(3S,4R)-3-(tert.-Butyl-dimethylsilyloxymethyl)-4-mercapto-2-oxo-azetidin-1-yl]-2-triphenylphosphoranyliden-essigsäure-allylesters werden in 20 ml abs. Methylenchlorid mit 1,7 ml Pyridin und anschliessend bei 0° tropfenweise mit einem Gemisch aus 2,47 g (2R,S)-2-Methyl-3-allyloxycarbonylamino-propionsäurechlorid und 10 ml abs. Methylenchlorid versetzt. Nach 30 Minuten Rühren wird der Feststoff über Hyflo abfiltriert und das Filtrat wird mit wässriger $NaHCO_3$-Lösung und dann mit Sole gewaschen. Nach Trocknen über $Na_2SO_4$ wird im Vakuum eingeengt. Der Rückstand wird durch Chromatographie an Silicagel (Laufmittel Toluol/Aethylacetat 4:1) gereinigt. DC (Silicagel, Toluol/Aethylacetat 1:1): $R_f$ = 0,3; IR ($CH_2Cl_2$): 3440, 1750, 1715, 1680, 1610 $cm^{-1}$.

Das Ausgangsmaterial (2R,S)-2-Methyl-3-allyloxycarbonylamino-propionsäurechlorid kann wie folgt hergestellt werden:

ea. (2R,S)-2-Methyl-3-allyloxycarbonylamino-propionsäure

Zu einer Lösung von 10 g (2R,S)-3-Amino-2-methyl-propionsäure in 20 ml Wasser und 44 ml 5 N NaOH-Lösung wird bei 0° 11,2 ml Chlorameisensäureallylester tropfenweise zugegeben. Nach 15 Stunden Rühren bei Raumtemperatur wird wie in Beispiel 1a aufgearbeitet. IR ($CH_2Cl_2$): 3450; 1710; 1505; 1220 $cm^{-1}$.

eb. (2R,S)-2-Methyl-3-allyloxycarbonylamino-propionsäurechlorid

0,374 g (2R,S)-2-Methyl-3-allyloxycarbonylamino-propionsäure wird bei 0° mit 0,6 ml Thionylchlorid veresetzt. Das Gemisch wird dann bei gleicher Temperatur 2 Stunden unter Schutzgas gerührt. Anschliessend wird mit absolutem Toluol verdünnt und am Rotationsverdampfer eingeengt. IR ($CH_2Cl_2$): 3440, 1780, 1710, 1500, 1215 $cm^{-1}$.

Beispiel 15: (5R,6S)-2-[(2R,S)-1-Allyloxycarbonylamino-prop-2-yl]-6-hydroxymethyl-2-penem-3-carbonsäure allylester

Analog Beispiel 2 werden 138 mg (5R,6S)-2-[(2R,S)-1-Allyloxycarbonylamino-prop-2-yl]-6-(tert.-butyldimethylsilyloxymethyl)-2-penem-3-carbonsäure-allylester in die Titelverbindung überführt:
DC (Silicagel, Toluol/Aethylacetat 1:1): $R_f$ = 0,08;
IR ($CH_2Cl_2$): 3610, 3440, 1780, 1710 $cm^{-1}$.

Beispiel 16: (5R,6S)-2-[(2R,S)-1-Aminoprop-2-yl]-6-hydroxymethyl-2-penem-3-carbonsäure

Eine Lösung von 82 mg (5R,6S)-2-[(2R,S)-1-Allyloxycarbonylamino-prop-2-yl]-6-hydroxymethyl-2-penem-3-carbonsäure-allylester in 3,5 ml abs. THF wird bei -10° mit 9 mg Tetrakis-(triphenylphosphin)-palladium und anschliessend mit 0,17 ml Tributylzinnhydrid versetzt. Nach 20 Minuten Rühren bei -10° werden 0,037 ml Essigsäure zugegeben und das Reaktionsgemisch wird nach Entfernen des Kühlbades 30 Minuten weitergerührt. Nach Konzentrieren am Rotationsverdampfer wird der Rückstand in Wasser-Aethylacetat aufgenommen, die wässrige Phase abgetrennt und die organische Phase noch 3 mal mit Wasser extrahiert. Die vereinigten wässrigen Phasen werden nach kurzem Konzentrieren am Rotationsverdampfer lyophilisiert.
UV (Phosphatpuffer pH 7,4): $\lambda$ max = 301 nm.
Das Reaktionsprodukt ist mit dem in Beispiel 12 dargestellten identisch.

Beispiel 17: (5R,6S)-2-[(2R,S)-2-Aminoprop-1-yl]-6-hydroxymethyl-2-penem-3-carbonsäure-1-äthoxycarbonyloxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 15,0 ml Methylenchlorid getropft und von den ausgefallenen anorganischen Salzen abfiltriert. Die Methylenchloridlösung wird bis auf 2 ml eingeengt und bei 0° zu einer Lösung von 0,257 g (1 mMol) (5R,6S)-2-[(2R,S)-2-Aminoprop-1-yl]-6-hydroxymethyl-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): Absorptionsbanden bei 1790 und 1740 $cm^{-1}$.

Beispiel 18: (5R,6S)-2-[(2R,S)-2-Aminoprop-1-yl]-6-hydroxymethyl-2-penem-3-carbonsäure-pivaloyloxymethylester

0,6 g Natriumjodid werden in 2 ml Aceton gelöst und mit 0,15 ml Pivalinsäurechlormethylester versetzt. Das Gemisch wird bei Raumtemperatur während 3 Stunden gerührt und anschliessend auf 7,5 ml Methylenchlorid getropft. Die ausgefallenen anorganischen Salze werden abfiltriert. Die Methylenchloridlösung wird bis auf 1 ml eingeengt und zu einer Lösung von 0,1 g (0,4 mMol) (5R,6S)-2-[(2R,S)-2-Aminoprop-1-yl]-6-hydroxymethyl-2-penem-3-carbonsäure und 0,07 ml Diisopropyläthylamin in 4 ml N,N-Dimethylacetamid bei 0° gegeben. Dann wird während 3 Stunden bei 0° gerührt, anschliessend mit Aethylacetat verdünnt und dreimal mit Wasser gewaschen. Die organische Phase wird über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): Absorptionsbanden bei 1790 und 1730 $cm^{-1}$.

Beispiel 19: In analoger Weise, wie in den vorangehenden Beispielen beschrieben, erhält man folgende Verbindungen:

(5R,6S)-2-[(2R,S)-2-Aminobut-1-yl]-6-hydroxymethyl-2-penem-3-carbonsäure, UV (Phosphatpuffer pH 7,4): $\lambda_{max}$ = 302 nm,

(5R,6S)-2-[(2R,S)-1-Aminoprop-2-yl]-6-[(1R)-1-hydroxyäthyl]-2-penem-3-carbonsäure, UV (Phosphatpuffer pH 7,4): $\lambda_{max}$ = 303 nm,

(5R,6S)-2-[(1S)-1-Amino-but-1-yl]-6-[(1R)-1-hydroxyäthyl]-2-penem-3-carbonsäure, UV (Phosphatpuffer pH 7,4)$\lambda_{max}$: 308 nm;

(5R,6S)-2-[(1S)-1-Aminobut-1-yl]-6-hydroxymethyl-2-penem-3-carbonsäure, UV (Phosphatpuffer pH 7,4)$\lambda_{max}$: 307,5 nm;

(5R,6S)-2-[(1R)-1-Aminoäthyl]-6-hydroxymethyl-2-penem-3-carbonsäure UV (Phosphatpuffer pH 7,4)$\lambda_{max}$: 308 nm,

(5R,6S)-2-[(1S)-1-Aminoäthyl]-6-hydroxymethyl-2-penem-3-carbonsäure UV (Phosphatpuffer pH 7,4)$\lambda_{max}$: 307 nm,

(5R,6S)-2-(2-Amino-2-methyl-prop-1-yl)-6-hydroxymethyl-2-penem-3-carbonsäure-1-äthoxycarbonyloxyäthylester, IR-Spektrum ($CH_2Cl_2$): 1788 und 1738 $cm^{-1}$;

(5R,6S)-2-[(3R,S)-3-Amino-but-1-yl]-6-hydroxymethyl-2-penem-3-carbonsäure-1-äthoxycarbonyloxyäthylester, IR-Spektrum ($CH_2Cl_2$): 1790 und 1736 $cm^{-1}$;

(5R,6S)-2-[(2R,S)-1-Aminoprop-2-yl]-6-hydroxymethyl-2-penem-3-carbonsäure-1-äthoxycarbonyloxyäthylester, IR-Spektrum ($CH_2Cl_2$): 1787 und 1735 $cm^{-1}$;

(5R,6S)-2-(2-Amino-2-methyl-prop-1-yl)-6-hydroxymethyl-2-penem-3-carbonsäure-pivaloyloxymethylester, IR-Spektrum ($CH_2Cl_2$): 1791 und 1733 $cm^{-1}$;

(5R,6S)-2-[(3R,S)-3-Amino-but-1-yl]-6-hydroxymethyl-2-penem-3-carbonsäure-pivaloyloxymethylester, IR-Spektrum ($CH_2Cl_2$): 1789 und 1732 $cm^{-1}$;
und

(5R,6S)-2-[(2R,S)-1-Aminoprop-2-yl]-6-hydroxymethyl-2-penem-3-carbonsäure-pivaloyloxymethylester, IR-Spektrum ($CH_2Cl_2$): 1788 und 1729 $cm^{-1}$.

Beispiel 20: (5R,6S)-2-[(1R)-1-Formamidinoäthyl]-6-hydroxymethyl-2-penem-3-carbonsäure

Eine Lösung von 110 mg Aethyl-formimidat-hydrochlorid und 84 mg Natriumhydrogencarbonat in 4 ml Wasser wird bei Raumtemperatur mit einer Lösung von 24 mg (5R,6S)-2-[(1R)-1-Aminoäthyl]-6-hydroxymethyl-2-penem-3-carbonsäure und 8,4 mg Natriumhydrogencarbonat in 1 ml Wasser versetzt. Nach 50 min. Rühren bei Raumtemperatur wird mit 1 ml 1N HCl versetzt und im Hochvakuum eingeengt. Die Rohsubstanz wird durch Chromatographie an OPTI UP $C_{12}$ gereinigt.
UV (Phosphatpuffer pH 7,4): $\lambda_{max}$: 305 nm.

Beispiel 21: Trockenampullen oder Vials, enthaltend 0,5 g (5R,6S)-2-[(2R,S)-2-Aminoprop-1-yl]-6-hydroxymethyl-2-penem-3-carbonsäure als Wirksubstanz werden wie folgt hergestellt:

Zusammensetzung (für 1 Ampulle oder Vial):

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele, wie z.B. die (5R,6S)-2-(2-Amino-2-methyl-prop-1-yl)-6-hydroxymethyl-2-penem-3-carbonsäure, die (5R,6S)-2-[(3R,S)-3-Amino-but-1-yl]-6-hydroxymethyl-2-penem-3-carbonsäure oder die (5R,6S)-2-[(2R,S)-1-Aminoprop-2-yl]-6-hydroxymethyl-2-penem-3-carbonsäure, verwendet werden.

Ansprüche (für alle Vertragsstaaten ausser AT)

1. Verbindungen der Formel

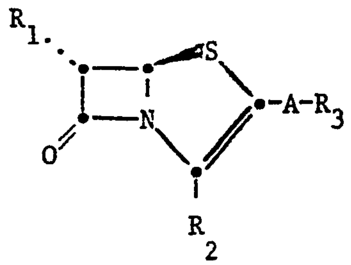

(I),

worin $R_1$ durch Hydroxy oder geschütztes Hydroxy substituiertes Niederalkyl ist, $R_2$ Carboxyl oder geschütztes Carboxyl $R_2'$ darstellt, $R_3$ Amino, durch Niederalkyl substituiertes Amino, substituiertes Methylenamino oder geschütztes Amino bedeutet und A durch Niederalkyl substituiertes geradkettiges Niederalkylen ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt, Salze von solchen Verbindungen der Formel I, die eine salzbildende Gruppe aufweisen, optische Isomere von Verbindungen der Formel I und Mischungen dieser optischen Isomere.

2. Verbindungen der Formel I gemäss Anspruch 1, worin $R_1$, $R_2$ und A die in Anspruch 1 angebenen Bedeutungen haben und $R_3$ Amino, substituiertes Methylenamino oder geschütztes Amino ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt, optische Isomere vor solchen Verbindungen, Mischungen dieser optischen Isomere, und Salze davon.

3. Verbindungen der Formel I gemäss Anspruch 1, worin $R_1$ durch Hydroxy oder geschütztes Hydroxy substituiertes Niederalkyl ist, $R_2$ Carboxyl oder geschütztes Carboxyl $R_2'$ bedeutet, $R_3$ Amino, Nie-

deralkylamino, Diniederalkylamino, eine Gruppe der Formel $-N=C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl, Pyridyl, z.B. 2- oder 4-Pyridyl, Thienyl, z.B. 2-Thienyl, oder Thiazolyl, z.B. 4-Thiazolyl ist, und $X_2$ Amino, Niederalkylamino, Diniederalkylamino, Niederalkylenamino, Hydrazino, Anilino, Niederalkoxy, Phenylniederalkoxy oder Niederalkylthio bedeutet, oder geschütztes Amino ist, und A durch Niederalkyl mit 1 bis 4 Kohlenstoffatomen substituiertes geradkettiges Niederalkylen darstellt, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt; Salze von solchen Verbindungen der Formel I, die eine salzbildende Gruppe enthalten, optische Isomere von Verbindungen der Formel I, welche in den Resten $R_1$ und/oder A Chiralitätszentren besitzen, und Mischungen dieser optischen Isomere.

4. Verbindungen der Formel I gemäss Anspruch 2, worin $R_1$, $R_2$ und A die in Anspruch 3 angegebenen Bedeutungen haben und $R_3$ Amino, eine Gruppe der Formel $-N=C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl, Pyridyl, z.B. 2- oder 4-Pyridyl, Thienyl, z.B. 2-Thienyl, oder Thiazolyl, z.B. 4-Thiazolyl ist, und $X_2$ Amino, Niederalkylamino, Diniederalkylamino, Niederalkylenamino, Hydrazino, Anilino, Niederalkoxy, Phenylniederalkoxy oder Niederalkylthio bedeutet, oder geschütztes Amino ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt.

5. Verbindungen der Formel I gemäss Anspruch 1, worin $R_1$ durch Hydroxy, Triniederalkylsilyloxy, 2-Halogenniederalkoxy, 2-Halogenniederalkoxycarbonyloxy oder gegebenenfalls durch Nitro substituiertes Phenylniederalkoxycarbonyloxy substituiertes Niederalkyl ist, $R_2$ Carboxyl, Niederalkenyloxycarbonyl, gegebenenfalls durch Nitro substituiertes Benzyloxycarbonyl, Niederalkanoylmethoxycarbonyl, 2-Halogenniederalkoxycarbonyl, 2-Triniederalkylsilyläthoxycarbonyl oder eine unter physiologischen Bedingungen spaltbare veresterte Carboxylgruppe, z.B. 1-Niederalkoxycarbonyloxyniederalkoxycarbonyl, Niederalkanoyloxymethoxycarbonyl, α-Aminoniederalkanoyloxymethoxycarbonyl oder Phthalidyloxycarbonyl, bedeutet, $R_3$ Amino, Niederalkylamino, Diniederalkylamino, eine Gruppe der Formel $-N=C(X_1,X_2)$, worin $X_1$ Wasserstoff, Amino, Niederalkylamino, Niederalkyl, Phenyl oder Pyridyl, z.B. 2-Pyridyl, und $X_2$ Amino, Niederalkylamino oder Diniederalkylamino ist; Azido, Phthalimino, Nitro, Niederalkenyloxycarbonylamino, gegebenenfalls durch Nitro substituiertes Benzyloxycarbonylamino, 1-Niederalkanoyl-niederalk-1-en-2-ylamino oder 1-Niederalkoxycarbonyl-niederalk-1-en-2-ylamino bedeutet, und A durch Niederalkyl mit 1 bis 4 Kohlenstoffatomen mono- oder disubstituiertes geradkettiges Niederalkylen darstellt, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt; Salze, von solchen Verbindungen der Formel I, die eine salzbildende Gruppe aufweisen, optische Isomere von Verbindungen der Formel I, welche in den Resten $R_1$ und/oder A Chiralitätszentren besitzen, und Mischungen dieser optischen Isomere.

6. Verbindungen der Formel I gemäss Anspruch 2, worin $R_1$, $R_2$ und A die in Anspruch 5 angegebenen Bedeutungen haben und $R_3$ Amino, eine Gruppe der Formel $-N=C(X_1,X_2)$, worin $X_1$ Wasserstoff, Amino, Niederalkylamino, Niederalkyl, Phenyl oder Pyridyl, z.B. 2-Pyridyl, und $X_2$ Amino, Niederalkylamino oder Diniederalkylamino ist; Azido, Phthalimido, Nitro, Niederalkenyloxycarbonylamino, gegebenenfalls durch Nitro substituiertes Benzyloxycarbonylamino, 1-Niederalkanoyl-niederalk-1-en-2-ylamino oder 1-Niederalkoxycarbonyl-niederalk-1-en-2-ylamino bedeutet, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt.

7. Verbindungen der Formel I gemäss Anspruch 1, worin $R_1$ durch Hydroxy substituiertes Niederalkyl und A durch Methyl oder Aethyl monosubstituiertes geradkettiges Niederalkylen mit 1 bis 4 Kohlenstoffatomen bedeutet oder worin $R_1$ Hydroxymethyl ist und A durch Methyl oder Aethyl disubstituiertes geradkettiges Niederalkylen mit 1 bis 4 Kohlenstoffatomen bedeutet, und worin $R_2$ jeweils Carboxyl, 1-Niederalkoxycarbonyloxyniederalkoxycarbonyl oder Niederalkanoyloxymethoxycarbonyl und $R_3$ jeweils Amino, Niederalkylamino oder Formamidino darstellen, Salze von solchen Verbindungen der Formel I, optische Isomere von Verbindungen der Formel I, welche in den Resten $R_1$ und/oder A Chiralitätszentren besitzen, und Mischungen dieser optischen Isomere.

8. Verbindungen der Formel I gemäss Anspruch 2, worin $R_1$, $R_2$ und A die in Anspruch 7 angegebenen Bedeutungen haben und $R_3$ Amino oder Formamidino darstellt.

9. Verbindungen der Formel I gemäss einem der Ansprüche 1 oder 2, worin

$R_1$ Hydroxymethyl oder 1-Hydroxyäthyl bedeutet und A durch Methyl oder Aethyl monosubstituiertes Aethylen oder 1,3-Propylen darstellt oder worin $R_1$ Hydroxymethyl ist und A durch Methyl disubstituiertes Aethylen bedeutet, und worin $R_2$ jeweils Carboxyl und $R_3$ jeweils Amino darstellen, und Salze von solchen Verbindungen der Formel I.

10. Die reinen optischen Isomere von Verbindungen der Formel I gemäss Anspruch 1, welche in den Substituenten $R_1$ und/oder A weitere Chiralitätszentren besitzen, insbesondere das (1'R)-Isomere von Verbindungen der Formel I, worin $R_1$ 1'-Hydroxyäthyl ist, und Salze von solchen Verbindungen der Formel I.

11. Die reinen optischen Isomere von Verbindungen der Formel 1 gemäss Anspruch 2, welche in den Substituenten $R_1$ und/oder A weitere Chiralitätszentren besitzen, insbesondere das (1'R)-Isomere von Verbindungen der Formel I, worin $R_1$ 1'-Hydroxyäthyl ist, und Salze von solchen Verbindungen der Formel I.

12. Pharmazeutisch annehmbare Salze von Verbindungen der Formel I gemäss Anspruch 1.

13. Pharmazeutisch annehmbare Salze von Verbindungen der Formel I gemäss Anspruch 2.

14. Unter physiologischen Bedingungen spaltbare Ester von Verbindungen der Formel I gemäss Anspruch 1.

15. Unter physiologischen Bedingungen spaltbare Ester von Verbindungen der Formel I gemäss Anspruch 2.

16. (5R,6S)-2-[(2R,S)-2-Aminoprop-1-yl]-6-hydroxymethyl-2-penem-3-carbonsäure und pharmazeutisch annehmbare Salze davon gemäss Anspruch 2.

17. (5R,6S)-2-(2-Amino-2-methyl-prop-1-yl)-6-hydroxymethyl-2-penem-3-carbonsäure und pharmazeutisch annehmbare Salze davon gemäss Anspruch 2.

18. (5R,6S)-2-[(3R,S)-3-Amino-but-1-yl]-6-hydroxymethyl-2-penem-3-carbonsäure und pharmazeutisch annehmbare Salze davon gemäss Anspruch 2.

19. (5R,6S)-2-(1-Formamidinoäthyl)-6-hydroxymethyl-2-penem-3-carbonsäure und pharmazeutisch annehmbare Salze davon gemäss Anspruch 1.

20. (5R,6S)-2-[(1R)-1-Aminoäthyl]-6-hydroxymethyl-2-penem-2-carbonsäure und pharmazeutisch annehmbare Salze davon gemäss Anspruch 1.

21. (5R,6S)-2-[(1S)-1-Aminoäthyl]-6-hydroxymethyl-2-penem-3-carbonsäure und pharmazeutisch annehmbare Salze davon gemäss Anspruch 1.

22. (5R,6S)-2-[(2R,S)-2-Amino-but-1-yl]-6-hydroxymethyl-2-penem-3-carbonsäure und pharmazeutisch annehmbare Salze davon gemäss Anspruch 2.

23. Pharmazeutische Präparate enthaltend Verbindungen der Formel I gemäss Anspruch 1 oder pharmazeutisch annehmbare Salze von solchen Verbindungen.

24. Pharmazeutische Präparate enthaltend Verbindungen der Formel I gemäss Anspruch 2 oder pharmazeutisch annehmbare Salze von solchen Verbindungen.

25. Verwendung von Verbindungen der Formel I gemäss Anspruch 1 und pharmazeutisch annehmbaren Salzen von solchen Verbindungen zur Herstellung von pharmazeutischen Präparaten.

26. Verwendung von Verbindungen der Formel I gemäss Anspruch 2 und pharmazeutisch annehmbaren Salzen von solchen Verbindungen zur Herstellung von pharmazeutischen Präparaten.

27. Verbindungen der Formel I gemäss Anspruch 1 und pharmazeutisch annehmbare Salze von solchen Verbindungen als antibiotische Mittel.

28. Verbindungen der Formel I gemäss Anspruch 2 und pharmazeutisch annehmbare Salze von solchen Verbindungen als antibiotische Mittel.

29. Verbindungen der Formel I gemäss Anspruch 1 und pharmazeutisch annehmbare Salze von solchen Verbindungen zur Anwendung in einem Verfahren zur therapeutischen Behandlung des menschlichen oder tierischen Körpers.

30. Verbindungen der Formel I gemäss Anspruch 2 und pharmazeutisch annehmbare Salze von solchen Verbindungen zur Anwendung in einem Verfahren zur therapeutischen Behandlung des menschlichen oder tierischen Körpers.

31. Verfahren zur Herstellung von Verbindungen der Formel I gemäss Anspruch 1, ihrer Salze, ihrer optischen Isomere und von Mischungen ihrer optischen Isomere, dadurch gekennzeichnet, dass man

a) eine Ylid-Verbindung der Formel

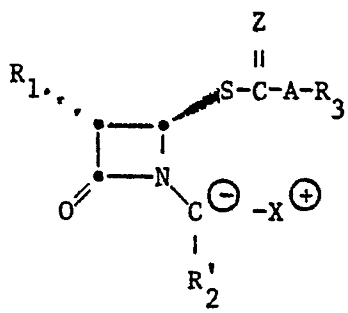

(II),

worin $R_1$, $R_2'$, $R_3$ und A die in Anspruch 1 angegebenen Bedeutungen

haben, wobei die Hydroxygruppe im Rest $R_1$ und eine freie Aminogruppe $R_3$ gegebenenfalls in geschützter Form vorliegen, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

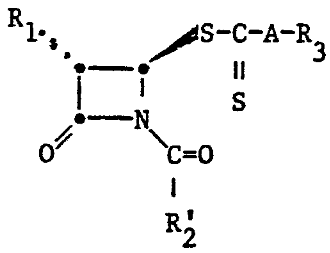

(III),

worin $R_1$, $R_2'$, $R_3$ und A die in Anspruch 1 angegebenen Bedeutungen haben, wobei die Hydroxygruppe im Rest $R_1$ und eine freie Aminogruppe $R_3$ gegebenenfalls in geschützter Form vorliegen, mit einer organischen Verbindung des dreiwertigen Phosphors behandelt, und wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_2'$ in die freie Carboxylgruppe oder in eine andere geschützte Carboxylgruppe $R_2'$ oder eine freie Carboxylgruppe $R_2$ in eine geschützte Carboxylgruppe $R_2'$ überführt, und/oder, wenn erwünscht, eine geschützte Aminogruppe $R_3$ in die freie Aminogruppe oder eine freie Aminogruppe $R_3$ in eine substituierte Aminogruppe überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

32. Verfahren zur Herstellung von Verbindungen der Formel I gemäss Anspruch 2, ihrer Salze, ihrer optischen Isomere und von Mischungen ihrer optischen Isomere, dadurch gekennzeichnet, dass man

a) eine Ylid-Verbindung der Formel (II), worin $R_1$, $R_2'$, $R_3$ und A die in Anspruch 2 angegebenen Bedeutungen haben, wobei die Hydroxygruppe im Rest $R_1$ und eine freie Aminogruppe $R_3$ gegebenenfalls in geschützter Form vorliegen, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel (III), worin $R_1$, $R_2'$, $R_3$ und A die in Anspruch 2 angegebenen Bedeutungen haben, wobei die Hydroxygruppe im Rest $R_1$ und eine freie Aminogruppe $R_3$ gegebenenfalls in geschützter Form vorliegen, mit einer organischen Verbindung des dreiwertigen Phosphors behandelt, und wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_2'$ in die freie Carboxylgruppe oder in eine andere geschützte Carboxylgruppe $R_2'$ oder eine freie Carboxylgruppe $R_2$ in eine geschützte Carboxylgruppe $R_2'$ überführt, und/oder, wenn erwünscht, eine geschützte Aminogruppe $R_3$ in die freie Aminogruppe oder eine freie Aminogruppe $R_3$ in eine substituierte Aminogruppe überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

33. Die nach dem Verfahren gemäss Anspruch 31 erhältlichen Verbindungen.

34. Die nach dem Verfahren gemäss Anspruch 32 erhältlichen Verbindungen.

FO 7.4 UL/we*/hc*

Ansprüche (für AT)

1. Verfahren zur Herstellung von Verbindungen der Formel

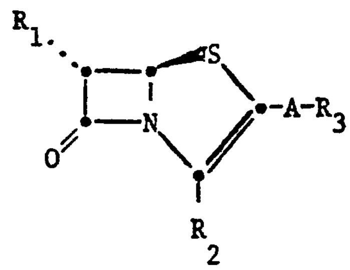

(I),

worin $R_1$ durch Hydroxy oder geschütztes Hydroxy substituiertes Niederalkyl ist, $R_2$ Carboxyl oder geschütztes Carboxyl $R_2'$ darstellt, $R_3$ Amino, durch Niederalkyl substituiertes Amino, substituiertes Methylenamino oder geschütztes Amino bedeutet und A durch Niederalkyl substituiertes geradkettiges Niederalkylen ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt, Salze von solchen Verbindungen der Formel I, die eine salzbildende Gruppe aufweisen, optische Isomere von Verbindungen der Formel I und Mischungen dieser optischen Isomere, dadurch gekennzeichnet, dass man

a) eine Ylid-Verbindung der Formel

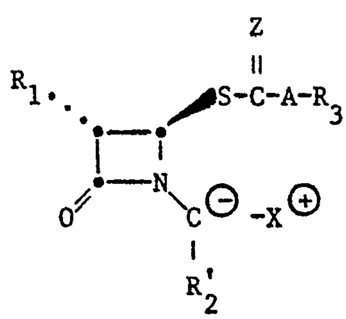

(II),

worin $R_1, R_2'$, $R_3$ und A die unter Formel I angegebenen Bedeutungen haben, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

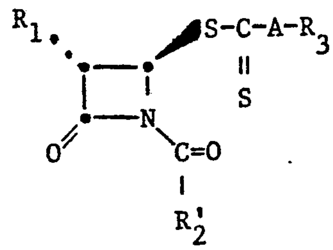

(III),

worin $R_1$, $R_2'$, $R_3$ und A die unter Formel I angegebenen Bedeutungen haben, mit einer organischen Verbindung des dreiwertigen Phosphors behandelt, und wenn erwünscht oder notwendig, in einer erhältlichen Verbindung der Formel I eine geschützte Hydroxygruppe im Rest $R_1$ in die freie Hydroxygruppe überführt, und/oder,wenn erwünscht, in einer erhältlichen Verbindung der Formel I eine geschützte Carboxylgruppe $R_2'$ in die freie Carboxylgruppe oder in eine andere geschützte Carboxylgruppe $R_2'$ oder eine freie Carboxylgruppe $R_2$ in eine geschützte Carboxylgruppe $R_2'$ überführt, und/oder, wenn erwünscht, eine geschützte Aminogruppe $R_3$ in die freie Aminogruppe oder eine freie Aminogruppe $R_3$ in eine substituierte Aminogruppe überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

2. Verfahren zur Herstellung von Verbindungen der Formel I gemäss Anspruch 1, worin $R_1$, $R_2$ und A die in Anspruch 1 angegebenen Bedeutungen haben und $R_3$ Amino, substituiertes Mehtylenamino oder geschütztes Amino ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt, optische Isomere vor solchen Verbindungen, Mischungen dieser optischen Isomere, und Salze davon, dadurch gekennzeichnet, dass man a) eine Ylid-Verbindung der Formel (II),worin $R_1$, $R_2'$, $R_3$ und A die oben angegebenen Bedeutungen

haben, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel (III), worin $R_1$, $R_2'$, $R_3$ und A die oben angegebenen Bedeutungen haben, mit einer organischen Verbindung des dreiwertigen Phosphors behandelt, und wenn erwünscht oder notwendig, in einer erhältlichen Verbindung der Formel I eine geschützte Hydroxygruppe im Rest $R_1$ in die freie Hydroxygruppe überführt, und/oder, wenn erwünscht, in einer erhältlichen Verbindung der Formel I eine geschützte Carboxylgruppe $R_2'$ in die freie Carboxylgruppe oder in eine andere geschützte Carboxylgruppe $R_2'$ oder eine freie Carboxylgruppe $R_2$ in eine geschützte Carboxylgruppe $R_2'$ überführt, und/oder, wenn erwünscht, eine geschützte Aminogruppe $R_3$ in die freie Aminogruppe oder eine freie Aminogruppe $R_3$ in eine substituierte Aminogruppe überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

3. Verfahren zur Herstellung von Verbindungen der Formel I gemäss Anspruch 1, worin $R_1$ durch Hydroxy oder geschütztes Hydroxy substituiertes Niederalkyl ist, $R_2$ Carboxyl oder geschütztes Carboxyl $R_2'$ bedeutet, $R_3$ Amino, Niederalkylamino, Diniederalkylamino, eine Gruppe der Formel $-N=C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl, Pyridyl, z.B. 2- oder 4-Pyridyl, Thienyl, z.B.

2-Thienyl, oder Thiazolyl, z.B. 4-Thiazolyl ist, und $X_2$ Amino, Niederalkylamino, Diniederalkylamino, Niederalkylenamino, Hydrazino, Anilino, Niederalkoxy, Phenylniederalkoxy oder Niederalkylthio bedeutet, oder geschütztes Amino ist, und A durch Niederalkyl mit 1 bis 4 Kohlenstoffatomen substituiertes geradkettiges Niederalkylen darstellt, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt; Salze von solchen Verbindungen der Formel I, die eine salzbildende Gruppe enthalten, optische Isomere von Verbindungen der Formel I, welche in den Resten $R_1$ und/oder A Chiralitätszentren besitzen, und Mischungen dieser optischen Isomere.

4. Verfahren zur Herstellung von Verbindungen der Formel I gemäss Anspruch 2, worin $R_1$, $R_2$ und A die in Anspruch 3 angegebenen Bedeutungen haben und $R_3$ Amino, eine Gruppe der Formel $-N=C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl, Pyridyl, z.B. 2- oder 4-Pyridyl, Thienyl, z.B. 2-Thienyl, oder Thiazolyl, z.B. 4-Thiazolyl ist, und $X_2$ Amino, Niederalkylamino, Diniederalkylamino, Niederalkylenamino, Hydrazino, Anilino, Niederalkoxy, Phenylniederalkoxy oder Niederalkylthio bedeutet, oder geschütztes Amino ist, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt.

5. Verfahren zur Herstellung von Verbindungen der Formel I gemäss Anspruch 1, worin

$R_1$ durch Hydroxy , Triniederalkylsilyloxy, 2-Halogenniederalkoxy, 2-Halogenniederalkoxycarbonyloxy oder gegebenenfalls durch Nitro substituiertes Phenylniederalkoxycarbonyloxy substituiertes Niederalkyl ist, $R_2$ Carboxyl, Niederalkenyloxycarbonyl, gegebenenfalls durch Nitro substituiertes Benzyloxycarbonyl, Niederalkanoylmethoxycarbonyl, 2-Halogenniederalkoxycarbonyl, 2-Triniederalkylsilyläthoxycarbonyl oder eine unter physiologischen Bedingungen spaltbare veresterte Carboxylgruppe, z.B. 1-Niederalkoxycarbonyloxyniederalkoxycarbonyl, Niederalkanoyloxymethoxycarbonyl, α-Aminoniederalkanoyloxymethoxycarbonyl oder Phthalidyloxycarbonyl, bedeutet, $R_3$ Amino, Niederalkylamino, Diniederalkylamino, eine Gruppe der Formel $-N=C(X_1,X_2)$, worin $X_1$ Wasserstoff, Amino, Niederalkylamino, Niederalkyl, Phenyl oder Pyridyl, z.B. 2-Pyridyl, und $X_2$ Amino, Niederalkylamino oder Diniederalkylamino ist; Azido, Phthalimino, Nitro, Niederalkenyloxycarbonylamino, gegebenenfalls durch Nitro substituiertes Benzyloxycarbonylamino, 1-Niederalkanoyl-niederalk-1-en-2-ylamino oder 1-Niederalkoxycarbonyl-niederalk-1-en-2-ylamino bedeutet, und A durch Niederalkyl mit 1 bis 4 Kohlenstoffatomen mono- oder disubstituiertes geradkettiges Niederalkylen darstellt, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt; Salze, von solchen Verbindungen der Formel I, die eine salzbildende Gruppe aufweisen, optische Isomere von Verbindungen der Formel I, welche in den Resten $R_1$ und/oder A Chiralitätszentren besitzen, und Mischungen dieser optischen Isomere.

6. Verfahren zur Herstellung von Verbindungen der Formel I gemäss Anspruch 2, worin $R_1$, $R_2$ und A die in Anspruch 5 angegebenen Bedeutungen haben und $R_3$ Amino, eine Gruppe der Formel $-N=C(X_1,X_2)$, worin $X_1$ Wasserstoff, Amino, Niederalkylamino, Niederalkyl, Phenyl oder Pyridyl, z.B. 2-Pyridyl, und $X_2$ Amino, Niederalkylamino oder Diniederalkylamino ist; Azido, Phthalimino, Nitro, Niederalkenyloxycarbonylamino, gegebenenfalls durch Nitro substituiertes Benzyloxycarbonylamino, 1-Niederalkanoyl-niederalk-1-en-2-ylamino oder 1-Niederalkoxycarbonyl-niederalk-1-en-2-ylamino bedeutet, mit der Massgabe, dass $R_1$ von 1-Hydroxyäthyl oder geschütztem 1-Hydroxyäthyl verschieden ist, wenn A durch zwei Methylgruppen geminal substituiertes geradkettiges Niederalkylen ist, $R_2$ obige Bedeutung hat und $R_3$ Amino oder geschütztes Amino darstellt.

7. Verfahren zur Herstellung von Verbindungen der Formel I gemäss Anspruch 1, worin $R_1$ durch Hydroxy substituiertes Niederalkyl und A durch Methyl oder Aethyl monosubstituiertes geradkettiges Niederalkylen mit 1 bis 4 Kohlenstoffatomen bedeutet oder worin $R_1$ Hydroxymethyl ist und A durch Methyl oder Aethyl disubstituiertes geradkettiges Niederalkylen mit 1 bis 4 Kohlenstoffatomen bedeutet, und worin $R_2$ jeweils Carboxyl, 1-Niederalkoxycarbonyloxyniederalkoxycarbonyl oder Niederalkanoyloxymethoxycarbonyl und $R_3$ jeweils Amino, Niederalkylamino oder Formamidino darstellen, Salze von solchen Verbindungen der Formel I, optische Isomere von Verbindungen der Formel I, welche in den Resten $R_1$ und/oder A Chiralitätszentren besitzen, und Mischungen dieser optischen Isomere.

8. Verfahren zur Herstellung von Verbindungen der Formel I gemäss Anspruch 2, worin $R_1$, $R_2$ und A die in Anspruch 7 angegebenen Bedeutungen haben und $R_3$ Amino oder Formamidino ist.

9. Verfahren zur Herstellung von Verbindungen der Formel I gemäss einem der Ansprüche 1 oder 2, worin $R_1$ Hydroxymethyl oder 1-Hydroxyäthyl bedeutet und A durch Methyl oder Aethyl monosubstituiertes Aethylen oder 1,3-Propylen darstellt oder worin $R_1$ Hydroxymethyl ist und A durch Methyl disubstituiertes Aethylen bedeutet, und worin $R_2$ jeweils Carboxyl und $R_3$ jeweils Amino darstellen, und Salze von solchen Verbindungen der Formel I.

10. Verfahren zur Herstellung der reinen optischen Isomere von Verbindungen der Formel I gemäss Anspruch 1, welche in den Substituenten $R_1$ und/oder A weitere Chiralitätszentren besitzen, insbesondere das (1'R)-Isomere von Verbindungen der Formel I, worin $R_1$ 1'-Hydroxyäthyl ist, und Salze von solchen Verbindungen der Formel I.

11. Verfahren zur Herstellung der reinen optischen Isomere von Verbindungen der Formel I gemäss Anspruch 2, welche in den Substituenten $R_1$ und/oder A weitere Chiralitätszentren besitzen, insbesondere das (1'R)-Isomere von Verbindungen der Formel I, worin $R_1$ 1'-Hydroxyäthyl ist, und Salze von solchen Verbindungen der Formel I.

12. Verfahren zur Herstellung von pharmazeutisch annehmbaren Salzen von Verbindungen der Formel I gemäss Anspruch 1.

13. Verfahren zur Herstellung von pharmazeutisch annehmbaren Salzen von Verbindungen der Formel I gemäss Anspruch 2.

14. Verfahren zur Herstellung von unter physiologischen Bedingungen spaltbaren Estern von Verbindungen der Formel I gemäss Anspruch 1.

15. Verfahren zur Herstellung von unter physiologischen Bedingungen spaltbaren Estern von Verbindungen der Formel I gemäss Anspruch 2.

16. Verfahren zur Herstellung von (5R,6S)-2-[2R,S)-2-Aminoprop-1-yl]-6-hydroxymethyl-2-penem-3-carbonsäure und pharmazeutisch annehmbaren Salzen davon gemäss Anspruch 2.

17. Verfahren zur Herstellung von (5R,6S)-2-(2-Amino-2-methyl-prop-1-yl)-6-hydroxymethyl-2-penem-3-carbonsäure und pharmazeutisch annehmbaren Salzen davon gemäss Anspruch 2.

18. Verfahren zur Herstellung von (5R,6S)-2-[(3R,S)-3-Amino-but-1-yl]-6-hydroxymethyl-2-penem-3-carbonsäure und pharmazeutisch annehmbaren Salzen davon gemäss Anspruch 2.

19. Verfahren zur Herstellung von (5R,6S)-2-(1-Formamidinoäthyl)-6-hydroxymethyl-2-penem-3-carbonsäure und pharmazeutisch annehmbaren Salzen davon gemäss Anspruch 1.

20. Verfahren zur Herstellung von (5R,6S)-2-[(1R)-1-Aminoäthyl]-6-hydroxymethyl-2-penem-3-carbonsäure und pharmazeutisch annehmbaren Salzen davon gemäss Anspruch 1.

21. Verfahren zur Herstellung von (5R,6S)-2-[(1S)-1-Aminoäthyl]-6-hydroxymethyl-2-penem-3-carbonsäure und pharmazeutisch annehmbaren Salzen davon gemäss Anspruch 1.

22. Verfahren zur Herstellung von (5R,6S)-2-[(2R,S)-2-Amino-but-1-yl]-6-hydroxymethyl-2-penem-3-carbonsäure und pharmazeutisch annehmbaren Salzen davon gemäss Anspruch 2.

23. Verfahren zur Herstellung von pharmazeutischen Präparaten, dadurch gekennzeichnet, dass man eine gemäss Anspruch 1 erhältliche Verbindung mit einem pharmazeutisch annehmbaren Trägerstoff mischt.

24. Verfahren zur Herstellung von pharmazeutischen Präparaten, dadurch gekennzeichnet, dass man eine gemäss Anspruch 2 erhältliche Verbindung mit einem pharmazeutisch annehmbaren Trägerstoff mischt.

FO 7.4/UL/we*/hc*

0125208
Nummer der Anmeldung

EP 84 81 0205

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X,Y | EP - A - 0 070 204 (SUMITOMO)<br>* Ansprüche 1-13 und Zusammenfassung *<br>-- | 1-28, 31,32 | C 07 D 499/00<br>A 61 K 31/43//<br>C 07 F 7/18<br>C 07 F 9/65<br>C 07 D 205/08 |
| Y | EP - A - 0 002 210 (MERCK)<br>* Ansprüche *<br>-- | 1-15, 23-28, 31,32 | |
| A | EP - A - 0 003 960 (CIBA-GEIGY)<br>* Ansprüche 1-11 *<br>--<br>./. | 1-15, 23-28, 31,32 | **RECHERCHIERTE SACHGEBIETE (Int. Cl. ³)**<br>C 07 D 499/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-28,31-34
Unvollständig recherchierte Patentansprüche:
Nicht recherchierte Patentansprüche: 29,30
Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 06-07-1984 | CHOULY |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82

Europäisches Patentamt

**EUROPÄISCHER TEILRECHERCHENBERICHT**

0125208
Nummer der Anmeldung

-2- EP 84 81 02

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 2 950 898 (BRISTOL-MYERS) | |
| | * Ansprüche 1-5,10,11 * | 1-15, 23-28, 31,32 |
| | -- | |
| A | CHEMICAL ABSTRACTS, Band 96, Heft 17, 26. April 1982, Seite 729, Zusammenfassung Nr. 142565u COLUMBUS OHIO (US) | |
| | & JP - A - 81 166 194 (SANKYO CO., LTD.)(21-12-1981) | |
| | * Zusammenfassung * | 1,23, 31 |
| | -- | |
| A | GB - A - 2 078 220 (SANKYO) | |
| | * Ansprüche * | 1,23, 31 |
| | ---- | |

KLASSIFIKATION DER ANMELDUNG (Int Cl.³)

RECHERCHIERTE SACHGEBIETE (Int. Cl.³)